# EUROPEAN PATENT APPLICATION

(11) **EP 2 660 310 A1**
(43) Date of publication of application: **06.11.2013**
(21) Application number: 11853383.5
(22) Date of filing: 28.12.2011
(51) Int. Cl.: C12M 1/00, C12Q 1/68, G06N 3/00

(54) **COMPREHENSIVE GLAUCOMA DETERMINATION METHOD UTILIZING GLAUCOMA DIAGNOSIS CHIP AND DEFORMED PROTEOMICS CLUSTER ANALYSIS**

(30) Priority: 28.12.2010 JP 2010294176
(71) Applicant: Tashiro, Kei, Kyoto-shi, Kyoto 603-8162 (JP); Kinoshita, Shigeru, Osaka 545-0035 (JP); Santen Pharmaceutical Co., Ltd, Osaka-shi Osaka 533-8651 (JP)
(72) Inventor: YAGI, Tomohito, Kyoto-shi Kyoto 604-0907 (JP); NAKANO, Masakazu, Yokohama-shi Kanagawa 236-0017 (JP); MORI, Kazuhiko, Kyoto-shi Kyoto 606-0014 (JP); IKEDA, Yoko, Kyoto-shi Kyoto 601-8011 (JP); UENO, Morio, Kyoto-shi Kyoto 606-8336 (JP); TOKUDA, Yuichi, Kyoto-shi Kyoto 606-0831 (JP); YAGI, Katsumi, Toyonaka-shi Osaka 560-0054 (JP); YOSHII, Kengo, Osaka 589-0006 (JP); FUWA, Masahiro, Ikoma-shi Nara 630-0101 (JP)
(74) Representative: Gulde Hengelhaupt Ziebig & Schneider
(86) International application number: PCT/JP2011/080393
(87) International publication number: WO 2012/091093

(57) **Abstract**

A technique is provided for determining an attribute of a physiological condition of a mammal with a high degree of accuracy, including onset and progression of a human glaucoma. A determination result of genotype data and determination result of a determination of cytokine data are integrated in an integrated determining unit (114); a comparison is made between a number of case determinations and a number of control determinations so as to determine which is larger (S330); and determination is made that there is a Case (glaucoma) when the number of case determinations is larger than the number of control determinations or there is a Control (healthy individual) when the number of control determinations is larger than the number of case determinations.

## Description

### Technical Field

The present invention relates to a apparatus for discriminating an attribute of a physiological condition of a mammalian individual, a method for discriminating the attribute of a physiological condition of a mammalian individual, a apparatus for generating a discriminator employed for such a method, and a program for discriminating the attribute of a physiological condition of a mammalian individual.

### Background Art

Glaucoma is a disease that causes characteristic optic nerve cupping and impairment in a visual field by retinal ganglion cell death. An elevation in an intraocular pressure is thought to be a major cause for the nerve cupping and the impairment in the visual field in glaucoma. On the other hand, while there are also glaucomas wherein the intraocular pressure remains within a statistically calculated normal range, even in such a case, it is thought that a glaucoma develops because the intraocular pressure is at a sufficiently high level for causing the impairment in the visual field for an individual.

The basic treatment for glaucoma is to maintain low intraocular pressure. In order to maintain low intraocular pressure, it is necessary to consider the causes for high intraocular pressure. Therefore, in the diagnosis of glaucoma, it is important to classify the type of glaucoma according to the level of intraocular pressure and a cause thereof. As a cause of the intraocular pressure increase, the presence or absence of angle closure is important because it is a major drainage pathway for an aqueous humor filling the eye. Based on these perspectives, the primary glaucoma is broadly classified into two groups: a closed-angle glaucoma with accompanying angle closure, and an open-angle glaucoma without accompanying angle closure. Of these two groups of glaucomas, the open-angle glaucoma is further classified into a primary open-angle glaucoma, that is an open-angle glaucoma in a narrow sense with accompanying intraocular pressure increase, and a normal-tension glaucoma wherein an intraocular pressure is maintained within a normal range.

It has been long established that inheritance is involved in glaucoma. There is a report describing that 5% to 50% of open-angle glaucoma patients have a family history and it is generally understood that 20% to 25% of the cases have hereditary causes. Based on these reports, studies have been conducted to search for genes responsible for glaucoma. As a result, it has been reported that a mutation in a myocilin (MYOC) gene is associated with the open-angle glaucoma (see, Japanese Patent Application Laid-Open Publication No. 2002-306165 (hereinafter, referred to as "Patent Literature 1")), and that a mutation in optineurin gene (OPTN) is associated with normal tension glaucoma (see, Rezaie T, Child A, Hitchings A, et al. Adult-onset primary open-angle glaucoma caused by mutations in optineurin. Science. 2006;295(5557):1077-1079 (hereinafter, referred to as "Non Patent Literature 1")).

On the other hand, a single nucleotide polymorphism ("SNP", or "SNPs" for the plural form) is a substitution mutation wherein a single base is replaced by another base in a genomic base sequence of an individual. A SNP generally exists at a frequency of around 1% or higher in a population of an individual species. A SNP can be found in introns or exons, or in any other genomic region of a gene.

Several studies have been conducted on a relationship between SNP and glaucoma. For example, in WO 2008/130008 (hereinafter, referred to as "Patent Literature 2"), a known polymorphic site on a genome (autosome) is comprehensively analyzed for glaucoma patients and for non-patients without a family history of glaucoma. Patent Literature 2 describes that SNPs related to the onset of glaucoma have been found. In WO 2008/130009 (hereinafter, referred to as "Patent Literature 3"), a known polymorphic site on a genome from rapid progression glaucoma patients and a genome from slow progression glaucoma patients are comprehensively analyzed. Patent Literature 3 describes that SNPs related to the progression of glaucoma have been found.

Japanese Patent Application Laid-Open Publication No. 2010-94125 (hereinafter, referred to as "Patent Literature 4") describes that a phenotype manifesting a glaucoma, i.e., impairment of the peripheral retina, can be reproduced in a transgenic mouse expressing a variant of a mouse WDR36 polypeptide that introduces a mutation equivalent to the one that causes deletion of the 657th to 659th amino acid residues including a 658th aspartic acid residue in a human WDR36 polypeptide. Japanese Patent Application Laid-Open Publication No. 2010-115194 (hereinafter, referred to as "Patent Literature 5") describes that a known polymorphic site on a genome (particularly autosome) from glaucoma patients and non-patients are comprehensively analyzed and that SNPs related to the glaucoma have been found.

In Japanese Patent Application Laid-Open Publication (Translation of PCT Application) No. 2007-529218 (hereinafter, referred to as "Patent Literature 6"), several known and unknown SNPs are described as related to the onset of optic neuropathy, including glaucoma and Leber disease. In Japanese Patent Application Laid-Open Publication No. 2009-201385 (hereinafter, referred to as "Patent Literature 7"), genomic DNA from open-angle glaucoma (OAG) patients and genomic DNA from healthy individuals are compared. Patent Literature 7 describes a specific SNP for prostacyclin receptor (PTGIR) is very closely related to the onset of glaucoma.

On the other hand, several studies have been conducted even with respect to the relationship between protein expression levels and glaucoma. So far, methods have been described for diagnosis of glaucoma using an antibody that specifically recognizes a trabecular meshwork-induced glucocorticoid response (TIGR) protein, which is a glucocorticoid-induced protein produced by trabecular meshwork cells (Japanese Patent Application Laid-Open Publication (Translation of PCT Application) No. H10-509866 (hereinafter, referred to as "Patent Literature 8")), or quantitative determination of TGF-β in the aqueous humor (Min SH, Lee TI, Chung YS, Kim HK. Transforming growth factor-β levels in human aqueous humor of glaucomatous, diabetic and uveitic eyes. Korean J Ophthalmol. 2006;20(3):162-5(hereinafter, referred to as "Non Patent Literature 2")).

Japanese Patent Application Laid-Open Publication No. 2009-244125 (hereinafter, referred to as "Patent Literature 9") describes the discovery of a protein marker in blood that is specifically detected in glaucoma patients through a proteomic analysis of blood samples from patients with glaucoma and patients with another ophthalmic disease. There are also reports of various novel candidate markers found by a proteomic analysis of ocular tissues (Bhuattacharya SK, Crabb JS, Bonilha VL, Gu X, Takahara H, Crabb JW. Proteomics implicates peptidyl arginine deiminase 2 and optic nerve citrullimation in glaucoma pathogenesis. Invest Ophthalmol Vis Sci. 2006;47(6):2508-14 (hereinafter, referred to as "Non Patent Literature 3"); and Tezel G, Tang X, Cai J. Proteomic identification of oxidatively modified retinal proteins in a chronic pressure-induced rat model of glaucoma. Invest Ophthalmol Vis Sci. 2005;46(9):3177-87 (hereinafter, referred to as "Non Patent Literature 4")).

### Citation List

### Patent Literature

Patent Literature 1: Japanese Patent Application Laid-Open Publication No. 2002-306165;
Patent Literature 2: WO 2008/130008;
Patent Literature 3: WO 2008/130009;
Patent Literature 4: Japanese Patent Application Laid-Open Publication No. 2010-94125;
Patent Literature 5: Japanese Patent Application Laid-Open Publication No. 2010-115194;
Patent Literature 6: Japanese Patent Application Laid-Open Publication (Translation of PCT Application) No. 2007-529218;
Patent Literature 7: Japanese Patent Application Laid-Open Publication No. 2009-201385;
Patent Literature 8: Japanese Patent Application Laid-Open Publication (Translation of PCT Application) No. H10-509866; and
Patent Literature 9: Japanese Patent Application Laid-Open Publication No. 2009-244125.

### Non Patent Literature

Non Patent Literature 1: Rezaie T, Child A, Hitchings A, et al. Adult-onset primary open-angle glaucoma caused by mutations in optineurin. Science. 2006;295(5557):1077-1079;
Non Patent Literature 2: Min SH, Lee TI, Chung YS, Kim HK. Transforming growth factor-β levels in human aqueous humor of glaucomatous, diabetic and uveitic eyes. Korean J Ophthalmol. 2006;20(3):162-5;
Non Patent Literature 3: Bhuattacharya SK, Crabb JS, Bonilha VL, Gu X, Takahara H, Crabb JW. Proteomics implicates peptidyl arginine deiminase 2 and optic nerve citrullimation in glaucoma pathogenesis. Invest Ophthalmol Vis Sci. 2006;47(6):2508-14; and
Non Patent Literature 4: Tezel G, Tang X, Cai J. Proteomic identification of oxidatively modified retinal proteins in a chronic pressure-induced rat model of glaucoma. Invest Ophthalmol Vis Sci. 2005;46(9):3177-87.

### Summary of Invention

### Problems to be Resolved by the Invention

With regard to the following points, the conventional art disclosed in the above-described literature has potential for improvement.
First, explaining all genetic factors for glaucoma only by the genes disclosed in Patent Literature 1, Patent Literature 4, Patent Literature 7 and Non Patent Literature 1 is difficult, and thus the existence of an unknown glaucoma linked gene could have been predicted. Consequently, there is room for further improvement in the above-described conventional art with regard to an explanation of genetic factors involved in glaucoma.

Second, the conventional art disclosed in Patent Literature 2, Patent Literature 3, Patent Literature 5, and Patent Literature 6 only points out inherent factors such as SNP as a causative factor for glaucoma. However, there are also many other acquired factors that relate to glaucoma. Accordingly, there is room for further improvement in the above-described conventional art from a perspective of a precise determination for onset and progression of glaucoma.

Third, explaining all proteome level factors in glaucoma only by proteins disclosed in Patent Literature 8 and Non Patent Literature 2 is difficult, and thus the existence of an unknown glaucoma-linked protein is predicted. Therefore, there is room for further improvement in the above-described conventional art with regard to an explanation of the proteome level factors in glaucoma.

Fourth, in the conventional art disclosed in Patent Literature 9, Non Patent Literature 3 and Non Patent Literature 4, only the proteome level factors are listed as causative factors for glaucoma. However, there are also many other factors that relate to glaucoma. Accordingly, there is room for further improvement in the above-described conventional art from a perspective of a precise determination for onset, progression, and prognosis of glaucoma.

In light of the above-described considerations, an object of the present invention is to provide technology that precisely determines an attribute of a physiological condition of a mammal, including onset, infection, progression, and prognosis of various diseases.

### Means of Solving the Problem

According to the present invention, a apparatus for discriminating an individual attribute of a physiological condition of a mammalian individual has been provided. The apparatus comprises a learning data set aquiring unit for aquiring a learning data set, wherein the data set relates to a group of individuals consisting of prulal individuals used in the below-described machine learning, wherein the group of individuals is obtained from a parent population consisting of individuals belonging to the same species as the subject individual, and wherein the data set includes a combination of an attribute of a physiological condition of the individual, discrete data relating to a genomic base sequence of the individual, and contiguous data relating to an amount of a specific substance in the individual organism.

The apparatus also comprises a resampler that extracts a subdata set, wherein the subdata set relates to prulal different subgroups of individuals, wherein the subdata set is obtained by random resampling from the learning data set, and wherein the subdata set includes a combination of the attribute of a physiological condition of each individual included in the subgroups of the individuals, the discrete data relating to a genomic base sequence of each the individuals, and the contiguous data relating to an amount of a specific substance in each of the individual organisms.

The apparatus also comprises a first machine learning unit that learns a pattern of the attribute of a physiological condition and the discrete data of the individuals included in the prulal subdata sets by machine learning to obtain prulal first discriminators that differ from each other, the prulal discriminators discriminating the attribute of a physiological condition of each of the individuals included in the subdata set based on the discrete data. The apparatus also comprises a second machine learning unit that learns a pattern of the attribute of a physiological condition and the contiguous data included in the prulal subdata sets by machine learning to obtain prulal second discriminators that differ from each other, the prulal discriminators discriminating the attribute of a physiological condition of each of the individuals included in the subdata set based on the contiguous data.

The apparatus also comprises a subject data acquiring unit that acquires subject data consisting of the discrete data and the contiguous data relating to the subject individual including a combination of the discrete data relating to a genomic base sequence of the individual and the contiguous data relating to an amount of a specific substance in the individual organism, both of which are obtained from the subject individual. The apparatus also comprises a subject data analyzer that analyzes each the subject data by pattern analysis multiple times using the prulal first discriminators and second discriminators, and generates each of a first discrimination result and a second discrimination result of the attribute of physiological condition of the subject individual multiple times.

The apparatus also comprises an integrated determining unit that integrates the first discrimination result and the second discrimination result for each attribute of a physiological condition, and integrally determines the most frequently discriminated attribute of a physiological condition in the first discriminator and the second discriminator as the attribute of a physiological condition of the individual subject. The apparatus also comprises an outputting unit that outputs a result of the integrated determining unit.

According to the present configuration, prulal subdata sets are created that are different from each other, the prulal subdata sets constituting a part of the initially obtained learning data set. For each subdata set, two types of discriminators are created that are resulted from a machine learning of data from different viewpoints, including the discrete data relating to a genomic base sequence of prulal individuals constituting this subdata set, and the contiguous data relating to an amount of a specific substance in the prulal individual organisms. Using the two types of discriminators that are present for each of the prulal different subdata sets, a pattern analysis is performed on subject data that are separately acquired from subject individuals. As a result, two types of discrimination results are obtained for each of the prulal different subdata sets with respect to the separately acquired subject individuals, and these two types of discrimination results are subtotaled for each of the prulal different subdata sets. An attribute of a physiological condition of the largest combined value, which results from totaling and integrating the subtotal calculations by using a suitable calculation formula, is integrally determined as the attribute of a physiological condition of the individual subject. Therefore, an attribute of a physiological condition of a mammal is able to be precisely determined by this apparatus.

According to the present invention, a method for discriminating an individual attribute of a physiological condition of a mammalian individual has been provided. The method includes a step for acquiring a learning data set, wherein the data set relates to a group of individuals consisting of prulal individuals used in the below-described machine learning, wherein the group of individuals is obtained from a parent population consisting of individuals belonging to the same species as the subject individual, and wherein the data set includes a combination of an attribute of a physiological condition of the individual, discrete data relating to a genomic base sequence of the individual, and contiguous data relating to an amount of a specific substance in the individual organism.

The method also includes a step for extracting a subdata set, wherein the subdata set relates to prulal different subgroups of individuals, wherein the subdata set is obtained by random resampling from the learning data set, and wherein the subdata set includes a combination of the attribute of a physiological condition of each individual included in the subgroups of individuals, the discrete data relating to a genomic base sequence of each of the individuals, and the contiguous data relating to an amount of a specific substance in each of the individual organisms.

The method also includes a step for learning the pattern of the attribute of a physiological condition and the discrete data included in the prulal subdata sets by machine learning to obtain prulal first discriminators that differ from each other, wherein the prulal first discriminators are made for discriminating an attribute of a physiological condition of each individual included in the subdata set based on the discrete data. The method also includes a step for learning the pattern of the attribute of a physiological condition and the contiguous data included in the prulal subdata sets by machine learning to obtain prulal second discriminators that differ from each other, wherein the prulal second discriminators are made for discriminating an attribute of a physiological condition of each individual included in the subdata set based on the contiguous data.

The method also includes a step for acquiring subject data consisting of discrete data and the contiguous data relating to the subject individual including a combination of the discrete data relating to a genomic base sequence of the individual and the contiguous data relating to an amount of a specific substance in the individual organism, both of which are obtained from the subject individual. The method also includes a step for analyzing the pattern of the subject data multiple times using the prulal first discriminators and second discriminators each, and generates each of a first discrimination result and a second discrimination result of the attribute of physiological condition of the subject individual multiple times.

The method also includes a step for integrating the first discrimination result and the second discrimination result for each attribute of a physiological condition, and integrally determining the most frequently discriminated attribute of a physiological condition in the first discriminator and the second discriminator as the attribute of a physiological condition of the individual subject. The method also includes a step for outputting the result of the integrated determining unit.

According to the present method, prulal subdata sets are created that are different from each other, and the prulal subdata sets constitute a part of the initially obtained learning data set. For each subdata set, two types of discriminators are created; which result from the machine learning of data from different viewpoints. The two types of discriminators include: discrete data relating to a genomic base sequence of prulal individuals constituting this subdata set, and contiguous data relating to an amount of a specific substance in the prulal individual organisms. Using the two types of discriminators that are present for each of the prulal different subdata sets, the pattern analysis is done on subject data that is separately acquired from subject individuals. As a result, two types of discrimination results are obtained for each of the prulal different subdata sets with respect to the separately acquired subject individuals, and these two types of discrimination results are subtotaled for each of the prulal different subdata sets. An attribute of a physiological condition of the largest combined value, which results from totaling and integrating the subtotal calculations by using a suitable calculation formula, is integrally determined as the attribute of a physiological condition of the individual subject. Therefore, the physiological condition of a mammal can be precisely determined by this method.

According to the present invention, a apparatus is provided that generates a discriminator that is used for the above-described method. The apparatus comprises a learning data set acquiring unit that acquires a learning data set, wherein the data set relates to a group of individuals consisting of prulal individuals used in the below-described machine learning, wherein the group of individuals is obtained from a parent population consisting of individuals belonging to the same species as the subject individual, and wherein the data set includes a combination of an attribute of a physiological condition of the individual, discrete data relating to a genomic base sequence of the individual, and contiguous data relating to an amount of a specific substance in the individual organism.

The apparatus also comprises a resampler that extracts a subdata set, wherein the subdata set relates to prulal subgroups of individuals that differ from each other, wherein the subdata set is obtained by random resampling from the learning data set, and wherein the subdata set includes a combination of the attribute of a physiological condition of each individual included in the subgroups of individuals, the discrete data relating to a genomic base sequence of the each individual, and the contiguous data relating to an amount of a specific substance in the each individual organism.

The apparatus also comprises a first machine learning unit that learns the pattern of the attribute of a physiological condition and the discrete data included in the prulal subdata sets by machine learning to obtain prulal first discriminators that differ from each other, wherein the prulal first discriminators are made for discriminating the attribute of a physiological condition of each individual included in the subdata set based on the discrete data. The apparatus also comprises a second machine learning unit that learns the pattern of the attribute of a physiological condition and the contiguous data included in the prulal subdata sets by machine learning to obtain prulal second discriminators that differ from each other, the prulal second discriminators for discriminating the attribute of a physiological condition of each individual included in the subdata set based on the contiguous data. The apparatus also comprises an outputting unit that outputs the first discriminator and the second discriminator.

According to the present apparatus, prulal subdata sets are created that are different from each other, and the prulal subdata sets constitute a part of the initially obtained learning data set. For each subdata set, two types of discriminators are created that result from the machine learning of data from different viewpoints. The two types of discriminators include: discrete data relating to a genomic base sequence of prulal individuals constituting this subdata set, and contiguous data relating to an amount of a specific substance in the prulal individual organisms. Therefore, by the above-described method, a set of two types of discriminators are obtained that can precisely determine an attribute of a physiological condition of a mammal.

The present invention also provides separately a apparatus for discriminating an attribute of a physiological condition of a mammalian individual. The apparatus comprises a discriminator parameter acquiring unit that acquires the first discriminator parameter and the second discriminator parameter generated by the above-described apparatus.

The apparatus also comprises a subject data acquiring unit that acquires subject data consisting of discrete data and contiguous data relating to the subject individual including a combination of discrete data relating to a genomic base sequence of the individual and contiguous data relating to an amount of a specific substance in the individual, both of which are obtained from the subject individual. The apparatus also comprises a subject data analyzer that analyzes each of the patterns of the subject data multiple times using the prulal first discriminators and second discriminators, and generates each of the first discrimination result and the second discrimination result of the attribute of physiological condition of the subject individual multiple times.

The apparatus also comprises an integrated determining unit that integrates the first discrimination result and the second discrimination result for each attribute of a physiological condition, and integrally determines the most frequently discriminated attribute of a physiological condition in the first discriminator and the second discriminator as the attribute of a physiological condition of the individual subject. The apparatus also comprises an outputting unit that outputs a result of the integrated determining unit.

Two types of discriminators generated by the above-described apparatus are obtained by the apparatus, and the pattern analysis is performed with these two types of discriminators on the subject data on the subject individuals. As a result, the two types of discrimination results are obtained for each of the prulal different subdata sets with respect to the subject individuals, and these two types of discrimination results are subtotaled for each of the prulal different subdata sets. An attribute of a physiological condition of the largest combined value, which results from totaling and integrating the subtotal calculations by using a suitable calculation formula, is integrally determined as the attribute of a physiological condition of the individual subject. Therefore, the attribute of a physiological condition of a mammal is able to be precisely determined by this apparatus.

The above-described apparatus and method only represent a single embodiment of the present invention, and thus the apparatus and method of the present invention may also be any combination of the above-described components. A system, a computer program, a storage medium, and/or the like, of the present invention may also have the same configuration.

### Advantageous Effects of Invention

According to the present invention, an attribute of a physiological condition of a mammal can be precisely determined.

### Brief Description of Drawings

Fig. 1 is a schematic diagram describing an overview of a physiological condition discriminating apparatus according to the present embodiment;
Fig. 2 is a schematic diagram describing an overview of the physiological condition discriminating apparatus of the present embodiment;
Fig. 3 is a schematic diagram describing input and output of data through a physiological condition discriminating apparatus of the present embodiment
Fig. 4 is a functional block diagram for describing configuration of a physiological condition discriminating apparatus of the present embodiment;
Fig. 5 is a schematic diagram for describing a method of selecting a SNP on the basis of a result of a basic statistical analysis in the physiological condition discriminating apparatus of the present embodiment;
Fig. 6a is a schematic diagram for describing in detail a numerical formula used in normalization and a method that converts genotype data into a number that can be used in various analyses in the physiological condition discriminating apparatus of the present embodiment;
Fig. 6b is a schematic diagram for describing in detail a numerical formula used in normalization and a method that converts genotype data into a number that can be used in various analyses in the physiological condition discriminating apparatus of the present embodiment;
Fig. 7 is a functional block diagram for describing a configuration of a learning data set acquiring unit of a physiological condition discriminating apparatus of the present embodiment;
Fig. 8 is a functional block diagram for describing configuration of a resampler of the physiological condition discriminating apparatus according to the present embodiment;
Fig. 9a is visual data describing principles of principal component analysis used by the physiological condition discriminating apparatus of the present embodiment;
Fig. 9b is visual data describing principles of principal component analysis used by the physiological condition discriminating apparatus according to the present embodiment;
Fig. 10 is visual data describing a genotype data analysis example on the basis of principal component analysis used by the physiological condition discriminating apparatus of the present embodiment;
Fig. 11 is a schematic diagram describing principles of a discriminant analysis used by the physiological condition discriminating apparatus of the present embodiment;
Fig. 12 is visual data describing a genotype data analysis example on the basis of discriminant analysis used by a physiological condition discriminating apparatus of the present embodiment;
Fig. 13 is a schematic diagram describing principles of SVM used by the physiological condition discriminating apparatus of the present embodiment;
Fig. 14 is a schematic diagram describing principles of a genotype data analysis example on the basis of SVM used by the physiological condition discriminating apparatus of the present embodiment;
Fig. 15 is a functional block diagram describing a configuration of a first machine learning unit of the physiological condition discriminating apparatus of the present embodiment;
Fig. 16 is a schematic diagram for describing cytokine data used by the physiological condition discriminating apparatus of the present embodiment;
Fig. 17 is a schematic diagram for describing the cytokine data used by the physiological condition discriminating apparatus of the present embodiment;
Fig. 18 is a schematic diagram for describing the cytokine data used by the physiological condition discriminating apparatus of the present embodiment;
Fig. 19 is visual data describing a cytokine data analysis example on the basis of principal component analysis used by the physiological condition discriminating apparatus of the present embodiment;
Fig. 20 is visual data describing the cytokine data analysis example on the basis of discriminant analysis used by the physiological condition discriminating apparatus of the present embodiment;
Fig. 21 is visual data describing the cytokine data analysis example on the basis of SVM used by the physiological condition discriminating apparatus of the present embodiment;
Fig. 22 is a functional block diagram describing a configuration of a second machine learning unit of the physiological condition discriminating apparatus of the present embodiment;
Fig. 23 is a functional block diagram describing a configuration of a subject data acquiring unit of the physiological condition discriminating apparatus of the present embodiment;
Fig. 24 is a schematic diagram describing a configuration of an integrated determining unit of the physiological condition discriminating apparatus of the present embodiment;
Fig. 25 is visual data describing integration results of genotype data and cytokine data using an integrated determining unit of the physiological condition discriminating apparatus of the present embodiment;
Fig. 26 is visual data describing integration results of genotype data and cytokine data using an integrated determining unit of the physiological condition discriminating apparatus of the present embodiment;
Fig. 27 is a functional block diagram describing a configuration of a subject data analysis unit of the physiological condition discriminating apparatus of the present embodiment;
Fig. 28 is a functional block diagram describing a configuration of an integrated determining unit of the physiological condition discriminating apparatus of the present embodiment;
Fig. 29 is a functional block diagram describing a configuration of an outputting unit of the physiological condition discriminating apparatus of the present embodiment;
Fig. 30 is a flowchart describing a genotype data analysis operation of the physiological condition discriminating apparatus of the present embodiment;
Fig. 31 is a flowchart describing a cytokine data analysis operation of the physiological condition discriminating apparatus of the present embodiment;
Fig. 32 is a flowchart describing a subject data analysis operation of the physiological condition discriminating apparatus of the present embodiment; and
Fig. 33 is a functional block diagram for describing a modification of the present embodiment.

### Description of Embodiments

Hereinafter, an embodiment of the present invention will be explained with reference to the drawings. The same constituent elements are appended by the same reference signs, and thus the descriptions of these elements have also been omitted were applicable.

### Principles of a physiological condition discriminating apparatus

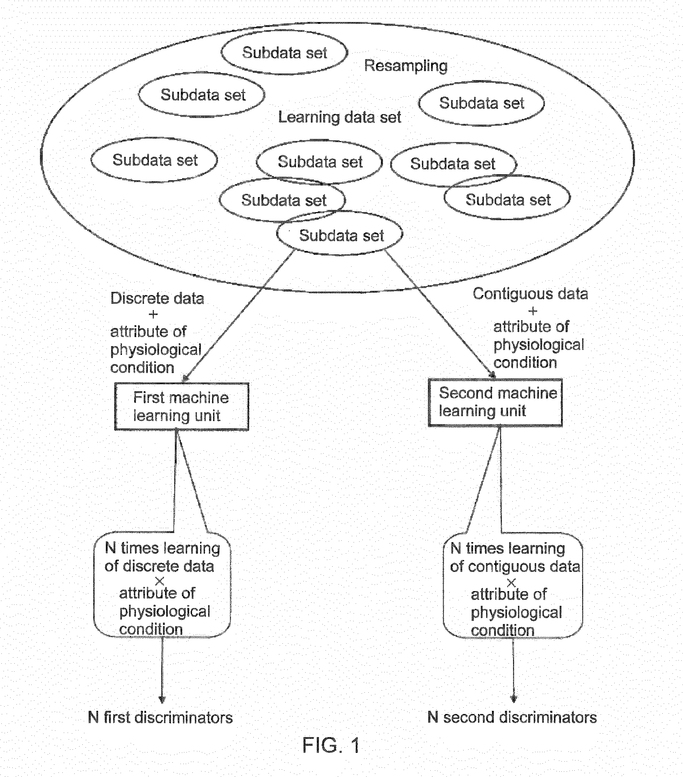

Fig. 1 is a schematic diagram describing an overview of the physiological condition discriminating apparatus of a present embodiment. In order to use the physiological condition discriminating apparatus, a learning data set acquired from a group of individuals including prulal individuals such as glaucoma patients and healthy individuals is first prepared. The learning data set includes a combination of discrete data relating to an attribute of a physiological condition (onset, progression, and prognosis of glaucoma, and / or the like), a genomic base sequence (a genotype constituted by an allele number of SNPs and/or the like), and contiguous data relating to an amount of a specific substance (such as blood cytokine concentration) in each individual organism. The prulal subdata sets resampled from the learning data set are prepared.

Next, machine learning such as primary component analysis, discriminant analysis, or support vector machine (SVM), is performed by inputting the prulal subdata sets into the first machine learning unit and the second machine learning unit, respectively. The first machine learning unit conducts the machine learning for relation between discrete data relating to a genomic base sequence and an attribute of a physiological condition of each individual, and the second machine learning unit conducts machine learning for relation between an amount of a specific substance and an attribute of a physiological condition of each individual. The machine learning is repeated N times (corresponding to the number of inputted subdata set) to obtain N first discriminators and N second discriminators.

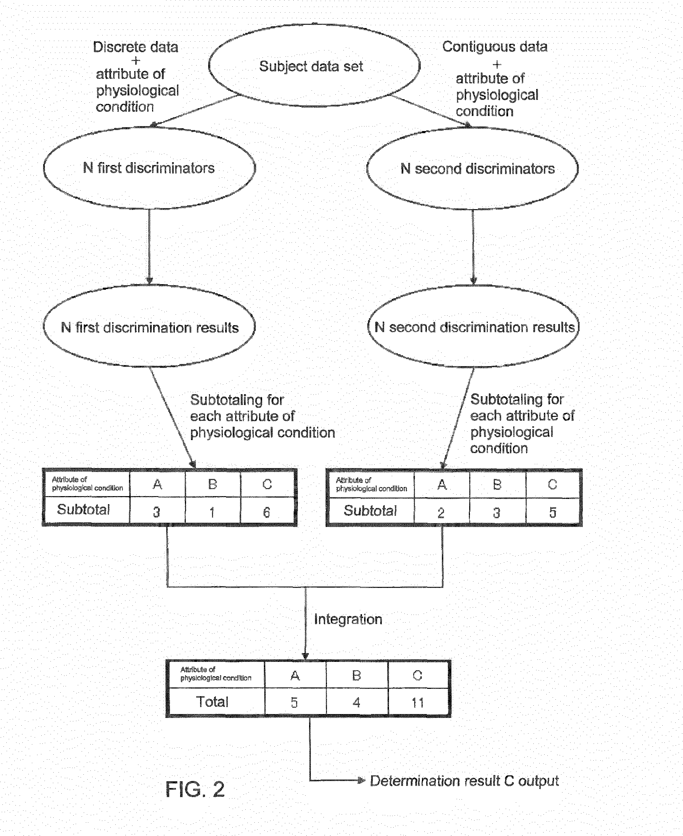

Fig. 2 is a schematic diagram describing an overview of the physiological condition discriminating apparatus of the present embodiment. Although Fig. 2 describes specific numerical examples along with the variable "N", the gist thereof is not specifically limited to these numerical examples. As in the description of Fig. 1, subject data is prepared for a subject with an unknown attribute of a physiological condition (such as a patient visiting a hospital with suspected glaucoma onset). This subject data includes a combination of the discrete data relating to a genomic base sequence (such as allele number in SNPs) of the individual and the contiguous data relating to an amount of a specific substance (such as blood cytokine concentration) in the individual organism, both of which are acquired from the subject individual.

The subject data is then analyzed with N first discriminators and N second discriminators obtained from the machine learning described in Fig. 1, and N first discrimination results and N second discrimination results are each acquired. These discrimination results determine an attribute (e.g., onset/normal, progressive/non-progressive, and favorable prognosis/unfavorable prognosis) of a physiological condition (e.g., onset, progression, and prognosis of glaucoma). Subsequently, the discrimination results are subtotaled for each attribute of a physiological condition. The sub-calculation results are integrated for each attribute of a physiological condition, and the integrated results are calculated. An attribute of a physiological condition with the highest number of determinations of the integration results is determined as the attribute of a physiological condition of the subject individual (such as a condition where a glaucoma is developing). As a result, in a case where a condition is determined to be a glaucoma that is developing, an operator who sees the determination result could advice the subject to seek a definitive diagnosis by an ophthalmologist. The definition of "progressive" and "non-progressive" in the context of an attribute of a physiological condition include the following meanings: "progressive" includes particularly rapid progression of a certain disease among affected individuals, while "non-progressive" includes not "progressive" case of a certain disease among affected individuals. In addition, an attribute of a physiological condition may be one other than that exemplified above, e.g., progressive/normal.

In order to construct a learning data set in the physiological condition discriminating apparatus of the present embodiment, an analysis resulting from a glaucoma diagnosis chip and/or the like may be suitably employed as the discrete data relating to a genomic base sequence of each individual. The glaucoma diagnosis chip is a custom DNA chip that is loaded with SNPs concerning glaucoma. As the contiguous data relating to the amount of a particular substance in each individual organism, analysis results of the comprehensive measurement of blood cytokine and/or the like may be suitably employed. Accordingly, the physiological condition discriminating apparatus of the present embodiment may be suitably employed in a presumptive diagnosis such as onset, progression and prognosis in glaucoma.

In order to develop a glaucoma diagnosis chip for acquiring the above-described discrete data, the present inventors obtained the candidate SNPs for a primary open-angle glaucoma (in broad terms) based on a extensive genome-wide association study, selected the optimal SNPs with a custom chip, determined the region with an LD block, and identified genes associated with the disease (Masakazu Nakano, et. al. Three susceptible loci associated with primary open-angle glaucoma identified by genome-wide association study in a Japanese population. Proc Natl Acad Sci. 2009;106(31):12838-12842). Similarly, the present inventors obtained the candidate SNPs for the primary open-angle glaucoma (in broad terms) based on an extensive genome-wide association study, and then also conducted an extensive genome/candidate gene association study on other ophthalmic diseases by utilizing the knowledge of this SNPs analysis. Consequently, the present inventors successfully developed the above-described glaucoma diagnosis chip with the aid of these study results. By using this glaucoma diagnosis chip, the physiological condition discriminating apparatus of the present embodiment may be suitably employed in a presumptive diagnosis such as onset, progression and prognosis in glaucoma.

On the other hand, in order to obtain the contiguous data described above, the present inventors learned a technique capable of precisely measuring various cytokine concentrations using a Cytometric Bead Array (CBA), which is a modified proteomics technique capable of measuring prulal cytokines simultaneously. Specifically, by measuring the concentrations of the prulal cytokines selected from the 29 cytokines described below and utilizing the results thereof as the above-described contiguous data, the physiological condition discriminating apparatus of the present embodiment may be suitably utilized in a presumptive diagnosis such as onset, progression and prognosis in glaucoma.

In other words, by integrating genotype data obtained with a DNA chip and blood cytokine data obtained with modified proteomics, the inventors developed an algorithm for conducting a presumptive diagnosis such as onset, progression and prognosis in glaucoma. During the study stage of this algorithm, the present inventors broadly applied various known statistical analysis, machine learning, and/or the like (primary component analysis, discriminant analysis, SVM, and/or the like), conducted selection of a useful technique, and ascertained data characteristics. The inventors then looked for an analysis technique that was effective for genotype data and cytokine data, respectively, and eventually integrated each result to examine a possibility for improving an overall diagnosis precision.

### General configuration

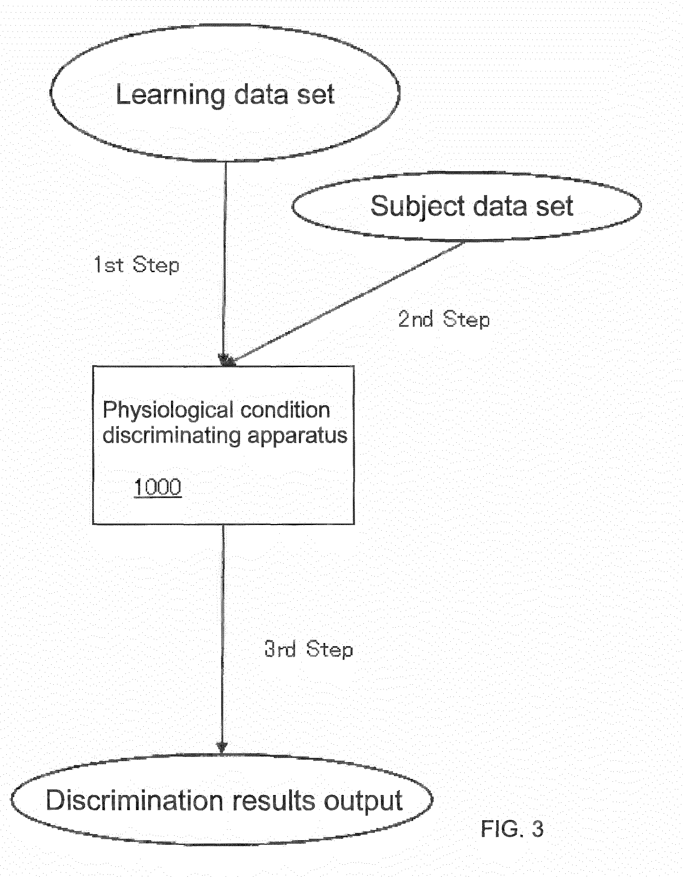

Fig. 3 is a schematic diagram describing the data input and output of the physiological condition discriminating apparatus 1000 of the present embodiment. As shown in this Figure, the physiological condition discriminating apparatus 1000 is configured to output the results of integrated determination when receiving an input of learning data set and subject data. The physiological condition discriminating apparatus 1000 is able to operate in such a manner because the physiological condition discriminating apparatus 1000 has a unique configuration as described below.

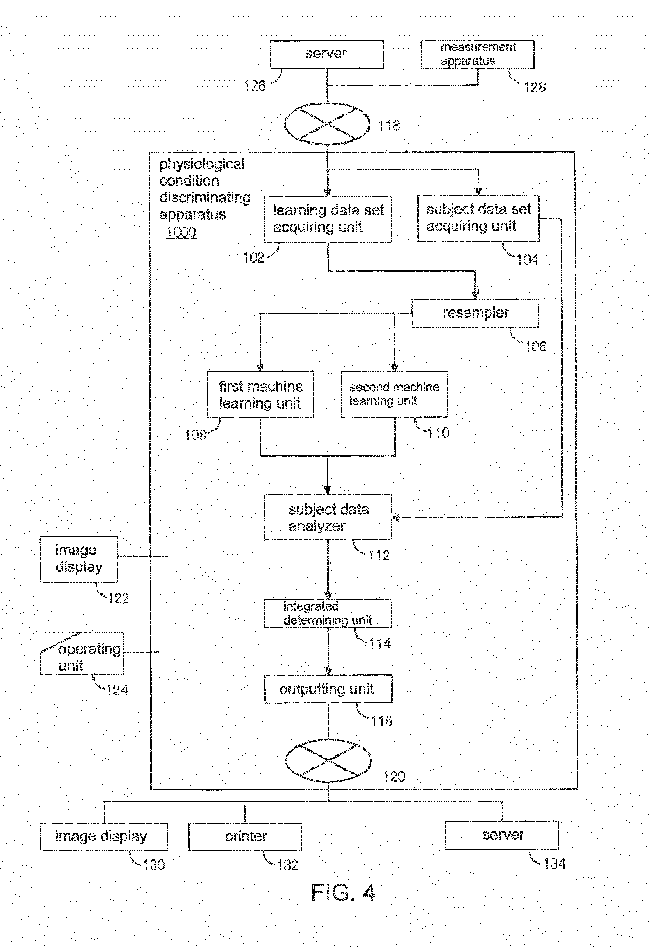

Fig. 4 is a functional block diagram describing the physiological condition discriminating apparatus 1000 of the present embodiment. The physiological condition discriminating apparatus 1000 is a apparatus for discriminating the attribute of a physiological condition such as the onset, progression, and prognosis of glaucoma in mammals including human.

The physiological condition discriminating apparatus 1000 comprises a learning data set acquiring unit 102 that acquires a learning data set relating to a group of individuals consisting of prulal individuals used in the below-described machine learning, wherein the group of individuals are obtained from a parent population consisting of individuals belonging to the same species as the subject individual. The parent population data set includes a combination of an attribute of a physiological condition of the individual, discrete data relating to a genomic base sequence of the individual, and contiguous data relating to an amount of a specific substance in the individual organism.

The physiological condition discriminating apparatus 1000 comprises a resampler 106, that extracts from the above-described learning data set, a subdata set relating to prulal subgroups that differ from each other, the subdata set constituting a part of the group of individuals. This subdata set includes a combination of an attribute of a physiological condition of each individual included in the subgroups of individuals, discrete data relating to a genomic base sequence of the each individual, and contiguous data relating to an amount of a specific substance in the each individual organism.

The physiological condition discriminating apparatus 1000 also comprises a first machine learning unit 108 that learns a pattern of the attribute of a physiological condition and the discrete data included in the above-described prulal subdata sets by machine learning. The first machine learning unit 108 is configured to obtain prulal first discriminators that differ from each other in order to discriminate the attribute of a physiological condition of each individual included in the prulal subdata sets based on the discrete data.

Similarly, the physiological condition discriminating apparatus 1000 also comprises a second machine learning unit 110 that learns a pattern of the attribute of a physiological condition and the contiguous data included in the above-described prulal subdata sets by machine learning. The second machine learning unit 110 is configured to obtain prulal second discriminators that differ from each other in order to discriminate the attribute of a physiological condition of each individual included in the prulal subdata sets based on contiguous data.

The physiological condition discriminating apparatus 1000 also comprises a data set acquiring unit 104 that acquires subject data consisting of discrete data and contiguous data relating to the individual subject. This subject data includes a combination of discrete data relating to a genomic base sequence of an individual and an amount of a specific substance in an individual organism. The subject data obtained by subject data set acquiring unit 104 is sent to the below-described subject data analyzer 112.

The physiological condition discriminating apparatus 1000 also comprises the subject data analyzer 112 that analyzes each of the patterns of the subject data multiple times using the prulal first discriminators and second discriminators. This data analyzer 112 is configured to generate each a first discrimination result and a second discrimination result of an attribute of physiological condition of the subject individual multiple times.

The physiological condition discriminating apparatus 1000 also comprises an integrated determining unit 114 that integrates the first discrimination result and the second discrimination result for each attribute of a physiological condition, and integrally determines the most frequently discriminated attribute of a physiological condition in the first discriminator and the second discriminator as an attribute of a physiological condition of the individual subject. The physiological condition discriminating apparatus 1000 comprises an outputting unit 116 that outputs a result of the integrated determining unit.

The physiological condition discriminating apparatus 1000 also comprises an operator 124 including a keyboard, a mouse and/or the like including a display 122 such as a liquid crystal display and/or the like. This allows a person operating the physiological condition discriminating apparatus 1000 to input various data or commands into the physiological condition discriminating apparatus 1000, while referencing to graphic data indicated on the display 122.

The physiological condition discriminating apparatus 1000 is also connected via a network 118 such as Internet, LAN, WAN, or VPN to a server 126 such as a file server, as well as a measuring apparatus 128 such as a DNA sequencer, a DNA chip, a PCR, an antibody chip or flow cytometry. This allows the physiological condition discriminating apparatus 1000 to read out the learning data set and subject data from the server 126, and to read learning data set and subject data directly from the measuring apparatus 128 as the measuring results.

The physiological condition discriminating apparatus 1000 is also connected via a network 118 such as the Internet, LAN, WAN, and VPN to a display 130 such as a liquid crystal display, a printer 132 such as a laser printer or an ink jet printer, and a server 134 such as a file server. This allows the physiological condition discriminating apparatus 1000 to display the results of integrated determination from outputting unit 116 on the display 130 as graphic data, to print it with the printer 132 as graphic data, and to let it be stored in the server 134 in various date formats.

According to the above-described unique configuration, the physiological condition discriminating apparatus 1000 is able to use the resampler 106 to create prulal subdata sets that are different from each other. The subdata sets constitute a part of the learning data set obtained via the learning data set acquiring unit 102. The physiological condition discriminating apparatus 1000 is also able to create two types of discriminators obtained by the first machine learning unit 108 and the second machine learning unit 110 that conduct a machine learning of data from different viewpoints for each subdata set. The two types of discriminators include: the discrete data relating to a genomic base sequence of prulal individuals constituting this subdata set, and the contiguous data relating to an amount of a specific substance in the prulal individual organisms.

The physiological condition discriminating apparatus 1000 can use these two types of discriminators for each of the prulal different subdata sets in the subject data analyzer 112 to perform the pattern analysis of the subject data on subject individuals acquired separately through a subject data set acquiring unit 104. As a result, two types of discrimination results are obtained for each of the prulal different subdata sets with respect to the separately acquired subject individuals, and these two types of discrimination results are subtotaled for each of the prulal the different subdata sets in the integrated determining unit 114. An attribute of a physiological condition of the largest combined value, which results from totaling and integrating the subtotal calculations by using a suitable calculation formula in the integrated determining unit 114, is integrally determined as the attribute of a physiological condition of the individual subject in the integrated determining unit 114.

The physiological condition discriminating apparatus 1000 outputs the integrated determination result from the outputting unit 116. Thus, the physiological condition discriminating apparatus 1000 is able to precisely determine an attribute of a physiological condition such as the onset, progression, and prognosis of glaucoma in mammals including a human.

### Discrete data

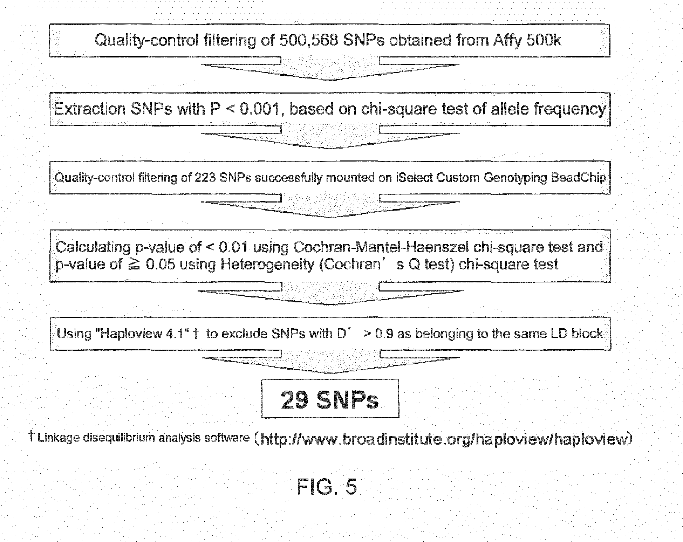

Fig. 5 is a schematic diagram that describes the genotype data used in a physiological condition discriminating apparatus of the present embodiment. As shown in this figure, data on a gene polymorphism or variant is used as the genotype data (discrete data relating to the genomic base sequence of the individual) for use in physiological condition discriminating apparatus of the present embodiment. As described in the Examples below, genotype data obtained by comprehensive examination of a genetic polymorphisms associated with attributes of the physiological condition can be used to improve the accuracy in determining the attribute of the physiological condition, including onset, progression and prognosis of glaucoma. A "gene polymorphism" in the present specification refers to a gene mutation that exists within a population at a frequency of at least 1%. On the other hand, a "variant" refers to a gene mutation that exists within a population at a frequency of less than 1%. Causes of gene polymorphisms or variants include various natural mutations occurring within a species, i.e. "substitutions", wherein a nucleotide is replaced by another nucleotide, "deletions", wherein a nucleotide is deleted, "insertions", wherein a nucleotide is inserted, "duplication", "genetic recombination", and/or the like. Among gene polymorphisms, a SNP having one nucleotide replaced by another nucleotide is considered as possessing an individualized marker for a genetic background.

This genotype data also concerns SNP. As described in the Examples below, a SNP is most efficiently and effectively used among the mammalian genetic polymorphisms on an attribute of a physiological condition such as the onset, progression and prognosis of glaucoma. Genotype data obtained by comprehensive examination of a SNP can further improve accuracy in determining the attribute of the physiological condition.

Specifically, in the present embodiment, as a first stage of the genotype data analysis, a genome analysis was conducted using a Genechip® Human Mapping 500k Array chip (Affy 500k) (Affymetrix, Inc.). As a second stage, reproducibly was confirmed by using a custom chip (iSelect) that employs a Select™ Custom Infinium™ Genotyping system while focusing on the SNPs that are significant in the first step.

Specifically, in the present embodiment, quality-control filtering of 500,568 SNPs obtained from an Affy 500k was performed, and the SNPs were narrowed down to 331,838 SNPs. An extraction of P < 0.001 was performed based on a chi-square test of allele frequency, and the SNPs were narrowed down to 255 SNPs. Among these, quality-control filtering of 223 SNPs successfully mounted on an iSelect Custom Genotyping BeadChip was performed, and the SNPs were narrowed down to 216 SNPs. A p-value of < 0.01 was extracted using Cochran-Mantel-Haenszel chi-square test, and a p-value of ≧ 0.05 was extracted using Heterogeneity (Cochran's Q test) chi-square test, and the SNPs were narrowed down to 40 SNPs. Finally, Haploview 4.1 was used as linkage disequilibrium analysis software to exclude SNPs with D' > 0.9 as belonging to the same LD block, 29 SNPs were ultimately selected as an analysis target.

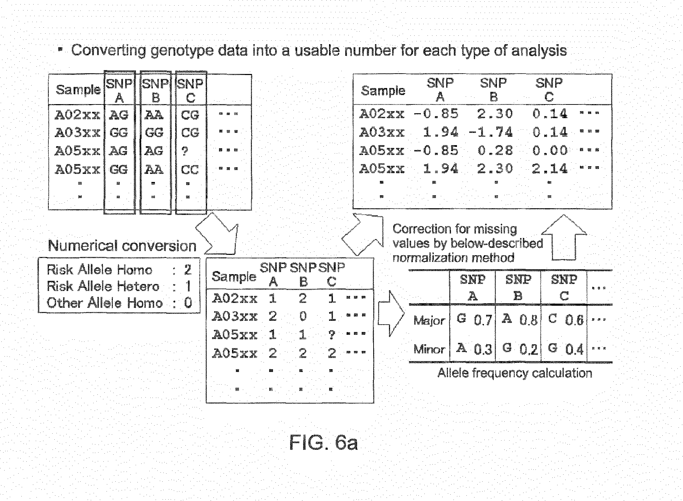

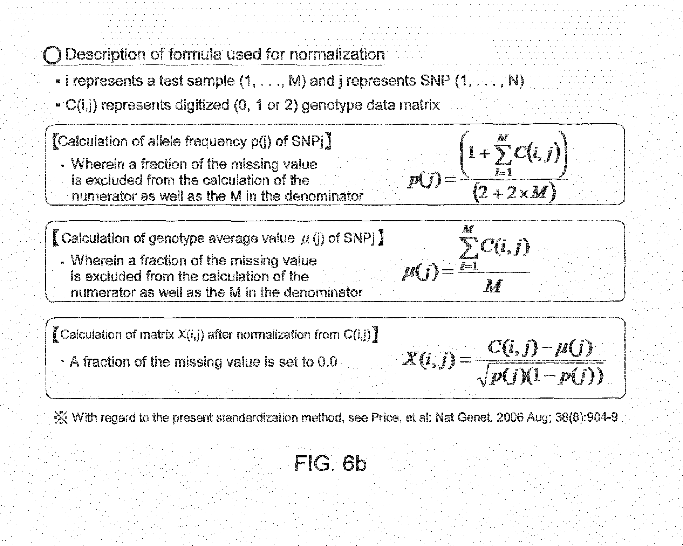

Fig. 6 is a schematic diagram for describing digitization of genotype data that is used in a physiological condition discriminating apparatus of the present embodiment. As indicated in the figure, the genotype data that is used in a physiological condition discriminating apparatus of the present embodiment is the one that is normalized for each individual based on the gene polymorphism or SNP allele frequency. As shown in this figure, this standardization technique is based on Price, et al: Nat Genet. 2006 Aug; 38(8):904-9. The method also allows for a correction for missing values. It is because by calculating a frequency of gene polymorphism or SNP allele and digitizing the frequency of occurrence of each allele, it is possible to quantitatively evaluate the extent at which the pattern of SNPs in the genome of the individual diverge from a typical pattern.

The genotype data is also data from an analysis result by a molecular biology method including a nucleic acid amplification method (e.g., TagMan PCR method, and RFLP), such as a DNA sequencer (including a next generation sequencer based on sequencing technology having a completely different principal than a Sanger method (1980 Nobel Prize in chemistry) and a conventional DNA sequencer based on the Sanger method), a DNA microarray, or a PCR method. When attempting to comprehensively examine the gene polymorphism or SNP in a genome-wide association, an examination utilizing these measuring apparatuss is advantageous from the perspective of efficiency, precision, and cost. An analysis result obtained from these measuring apparatuss may be read directly into the physiological condition discriminating apparatus 1000, or the result may be stored in, e.g., a server or a recording medium before being read into the physiological condition discriminating apparatus 1000. However, it is preferable to have the result stored in a server or a recording medium in order to accumulate and arrange genotype data from a large number of individuals for further utilization.

In this analysis of genotype data, genotype data is obtained in the above-described manner and suitable SNPs are selected first by the basic statistical analysis result. The genotype data obtained is digitized and a matrix of (sample number) x (SNP number) is created. Various analyses (primary component analysis, discriminant analysis, SVM, and/or the like) are conducted thereafter on this digitized genotype data matrix. For more details, refer to the description below.

### Learning data set acquiring unit

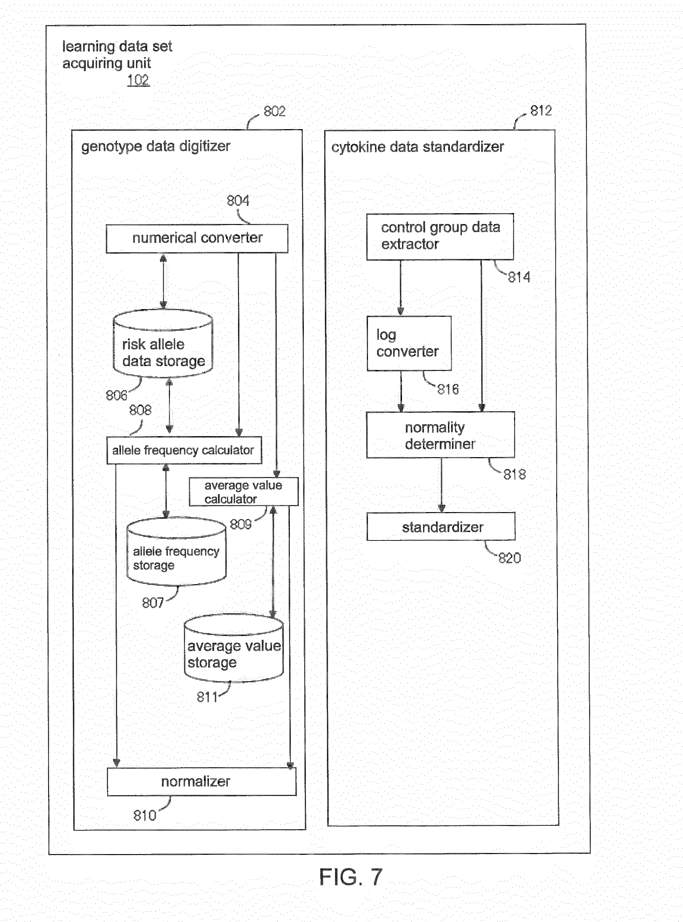

Fig. 7 is a functional block diagram for describing a configuration of the learning data set acquiring unit 102 of the physiological condition discriminating apparatus 1000 of the present embodiment. As indicated in the figure, the learning data set acquiring unit 102 comprises a genotype data digitizer 802 that converts the genotype data into digital data. This genotype data digitizer 802 comprises a numerical converter 804 that converts the acquired genotype data into a predetermined numerical value.

The digital converter 804 is connected to a risk allele data storage 806. This risk allele data storage 806 stores a risk allele database that includes relevant information on a risk allele and a non-risk allele. With reference to the genotype data and risk allele database, this numerical converter 804 assigns a numerical value in a given allele included in the genotype data, e.g., a numerical value 2 when the risk allele is homozygous, a numerical value 1 when the risk allele is heterozygous, and a numerical value 0 when a non-risk allele is homozygous. In this case, a correction for the missing value can be made by means of a normalization technique already described for Fig. 6.

The learning data set acquiring unit 102 comprises an allele frequency calculator 808 which calculates of the frequency of appearance of each allele in the genotype data included in the learning data set. The allele frequency calculator 808 calculates the allele frequency in each of the SNPs so that the total of the frequency of appearance of each allele is 1. The allele frequency calculator 808 also determines which allele in each of the SNPs is dominant. The frequency of appearance of each allele thus calculated is stored in an allele frequency storage 807 and this calculated frequency of appearance can be referred to from the outside when needed. The learning data set acquiring unit 102 also comprises an average value calculator 809 that calculates an average value of the appearance of each allele in the genotype data included in the learning data set. The frequency of appearance of each allele thus calculated is stored in an average value storage 809 and this calculated frequency of appearance can be referred to from the outside when needed. The learning data set acquiring unit 102 also comprises a normalizer 810 that normalizes the numerical data obtained by the numerical converter 804 based on the allele frequency calculated by the allele frequency calculator 808. As for the question of the definition of a risk allele, it is possible to determine a risk allele based on a difference in allele frequency, e.g., between an onset group and a control group or an onset group and non-onset group. Because the accuracy of allele frequency essentially increases along with the increase in total number of learning data sets used in the analysis, changes or revisions in the risk allele associated with a change in allele frequency are also possible when the learning data set acquires some change, revision, addition, and/or the like. While problems are unlikely to occur when the difference in the allele frequency is large, e.g., between 0.3 and 0.7, there is a possibility for the risk allele to be reversed along with a revision of a learning dataset when the difference is small, e.g., between 0.55 and 0.45. Accordingly, the allele frequency calculator 808 is configured so that the revision of the risk allele accompanying the revision of such a learning data set is possible.

As used herein, normalization includes transforming a non-normal form into a normal form (fixed form with a desirable property for an operation such as a comparison or calculation). There are various normalization methods including, e.g., a proportional transformation to make a root mean square equal to 1, and a linear transformation to make a mean equal to 0 and a variance equal to 1. Among the various normalization methods, the format of normalization means indicated in Fig. 6 is most preferable.

It is preferable that the genotype data used in the physiological condition discriminating apparatus 1000 of the present embodiment is data that is normalized for each individual with a normalizer 810 based on the allele frequency calculated in the allele frequency calculator 808, after a numerical transformation of the gene polymorphism or SNP allele in the numerical converter 804. By calculating the gene polymorphism or SNP allele frequency and digitizing the frequency of occurrence of each allele, it may be possible to quantitatively analyze the extent at which the pattern of SNPs in the genome of the individual diverge from a typical pattern.

As also indicated in the figure, the leaming data set acquiring unit 102 comprises a cytokine data standardizer 812 that transforms the cytokine data into standardized data. The cytokine data standardizer 812 comprises a control group data extractor 814 that extracts control group data (e.g., healthy individual data) from the cytokine data.

The control group data extractor 814 is connected to a Log converter that transforms the blood cytokine concentration for each type of cytokine into Log form. The Log converter 816 prepares the two types of values, i.e., the original value and the value that was transformed into Log form, only for the data of the each cytokine control group. The control group data extractor 814 and the Log converter 816 are connected to a normality determiner 818 that employs a value closer to a normal distribution by determining the normality of the original value and the Log value. The normality determiner 818 determines the normality in each of the original values and the Log transformed values, and individually determines values to be used based on each cytokine p-value.

As a verification of normality in the normality determiner 818, methods such as a comparison to a normal distribution curve, and an evaluation by kurtosis and skewness can be conveniently utilized. Such normality verification methods include, e.g., a test by skewness, a test by kurtosis, a test by skewness and kurtosis, a Kolmogorov-Smirnov test, and/or the like.

The normality determiner 818 is connected to the standardizer 820, which calculates an average value and standard deviation of the original value and the Log transformed value for the data of the control group only. It also performs standardization of all samples for each cytokine with the following equation.

Standardized value = (original value or Log transformed value - average value of control group) / (standard deviation of control group)

To obtain the cytokine data used in the physiological condition discriminating apparatus 1000 of the present embodiment, it is preferable to use a method such as CBA, which can measure a large number of cytokines simultaneously. However, there may be a change in a trend of values in some cases as a consequence of the combination of measurement items. In a method such as CBA, there may be cases where the range for possible values also changes due to a resetting of a standard curve for each measurement. Consequently, it is undesirable to make a simple comparison among the values obtained by measurement on different test days or under different test conditions, even for the same cytokines. For this reason, it is preferable not to use a concentration value from a measurement result as is. Instead, the result of the concentration measurement is standardized with a certain reference value that can be stably compared (e.g., control group data) in a unique standardization method that employs the control group as a reference.

In the physiological condition discriminating apparatus 1000 of the present embodiment, the learning data set acquiring unit 102 may be configured to read out the learning data set from a parent population database which stores the learning data set relating to a group of individuals and which may be located inside or outside the physiological condition discriminating apparatus 1000. For example, the learning data set acquiring unit 102 may be configured to read out the learning data set from the parent population database stored in a server 126 that is disposed in a facility such as a hospital through the network 118 such as Internet.

In this instance, the parent population database may be configured so that a combination of the attribute of a physiological condition of the new individual belongs to the same species as the subject individual, the discrete data relating to a genomic base sequence of the new individual, and the contiguous data relating to an amount of a specific substance in the new individual is added and updated as needed. In other words, the parent population database is stored in the server 126 located in the facility such as a hospital and configured to allow the genotype data, the cytokine data, and the confirmed diagnosis data acquired at the facility such as a hospital to be added and updated as needed.

### Resampling

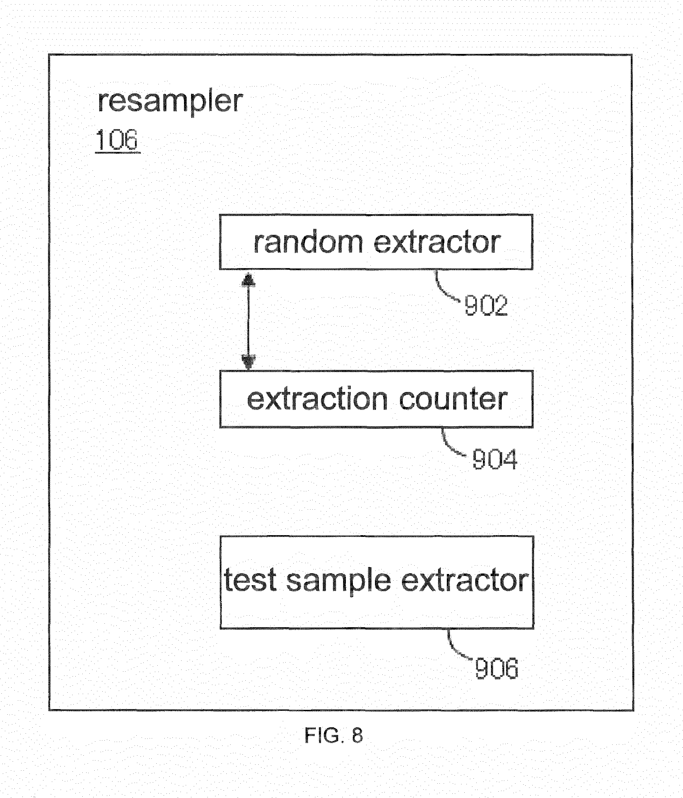

Fig. 8 is a functional block diagram for describing a configuration of the resampler 106 of the physiological condition discriminating apparatus 1000 of the present embodiment. As indicated in the figure, the resampler 106 comprises a random extractor 902 that randomly extracts the subdata set from the learning data set. Accordingly, the resampler 106 is capable of numerous random generations of subdata sets that include the data of the part of individuals from the learning data set including data of the prulal individuals. By using numerous random subdata sets, the below-described first machine learning unit 108 and the second machine learning unit 110 can conduct the machine learning, and the accuracy of the machine learning will be improved. Because there is a small possibility for the same subdata set to be generated by the resampler 106 in a random subdata set generation, the resampler may be configured to eliminate the duplication of the same subdata set in such cases.

The resampler 106 has an extraction counter 904 that controls an extraction process by a random extractor 902 to be repeated for a predetermined number of times (e.g., 10 times, 20 times, 30 times, 50 times, or 100 times) in response to the size of a learning data set. The resampler 106 is configured to perform extraction for the number of times appropriate for the size of the learning data set to be inputted. This number is not predetermined for the improved accuracy of the machine learning by the first machine learning unit 108 and the second machine learning unit 110 from a statistical point of view. The extraction counter 904 may also be configured to terminate the extraction process by the random extractor 902 when the discrimination accuracy exceeds the predetermined threshold value (or to terminates the extraction at the predetermined maximum extraction number when the threshold value cannot be reached). According to this resampler 106, it is possible to predetermine not only the number of resampling times but also the number of samples to be resampled. In this instance, the controller can be set to extract a certain number of samples (e.g., 10 samples, 20 samples, 30 samples, 50 samples, or 100 samples), which are predetermined according to the size of the learning data set. By controlling the number of extraction times and the number of extraction samples in this way, an optional resampling process could be possible, e.g., a resampling of 50 samples each out of 100 samples for 20 times.

The resampler 106 comprises an test sample extractor 906 for extracting test sample data. The test sample data is used in order to verify discrimination accuracy of an attribute of a physiological condition using the below-described first discriminator and second discriminator. Accordingly, the discrimination accuracy of the attribute of a physiological condition obtained from the below-described first discriminator and second discriminator can be verified with the test sample extractor 906. Consequently, it is possible to select an optimal analysis engine among the below-described analysis engines used in the first discriminator and the second discriminator such as principal component analysis engine, discriminant analysis engine, and SVM analysis engine. Using the test sample data generated by the test sample extractor 906, it is possible to optimize a weight parameter, which is applied to a subtotal result in the first discrimination result and the second discrimination result. The test sample data extracted by the test sample extractor 906 may also extract entire samples included in subdata set generated by the random extractor 902 as the test sample data for the learning by the first machine learning unit 108 and the second machine learning unit 110.

When a discrimination of an attribute of a physiological condition of a human disease such as glaucoma is attempted with the physiological condition discriminating apparatus 1000 of the present embodiment, improving the diagnostic capability using a limited data volume is a challenge because many samples can not be collected, in general, that have a complete set of the discrete data relating to a genomic base sequence of a individual and the contiguous data relating to an amount of a specific substance in an individual organism. The discrimination performance of the physiological condition discriminating apparatus 1000 of the present embodiment has been improved by creating many subdata sets by repeating the resampling, and individually analyzing these subdata sets to obtain multidirectional data in the resampler 106.

### First machine learning unit

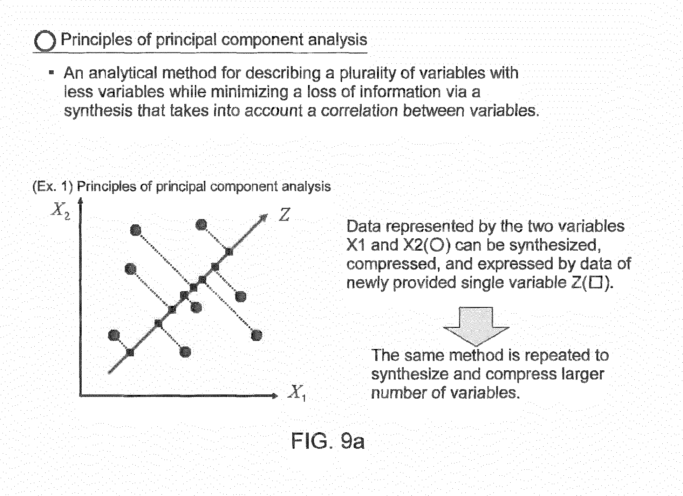

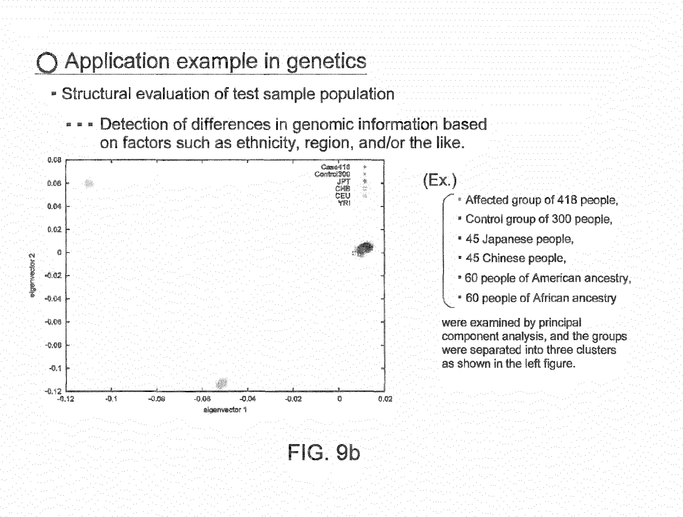

Fig. 9 shows visual data describing the principles of the principal component analysis used in the physiological condition discriminating apparatus of the present embodiment. The principal component analysis includes an analysis method that determines the overall properties of the multiple variables. The principal component analysis eliminates a correlation between the variables of quantitative data described by many variables and condenses the correlation with minimum information loss into a few uncorrelated composite variables for analysis. The method of principal component analysis was proposed by Hotelling around 1933 (from: Meitetsu Kin, Data Science by R, p. 66, published by Morikita). Among the many types of functions (analysis engines) used for the principal component analysis, the following may be preferably used: a method for direct determination of an eigenvector from "eigen" using "prcomp" and "princomp" written by a software "R" (a statistical analysis software that implements the R language) and a matrix calculation, which is a standard feature of "R"; and an eigenvector calculation by "LAPACK", which is a numerical calculation library for the C language and Fortran. In the field of genetics, principal component analysis is used in the structural evaluation of a population. As an example application in genetics, principal component analysis can be used for a structural evaluation of a sample population (detection of differences in genomic information due to a factor such as ethnicity, region, and/or the like). Specifically, when the principal component analysis is performed using two or three principal components on a population consisting of Africans, Europeans, and Asians, the principal component analysis is divided into three groups as can be seen in Fig. 9.

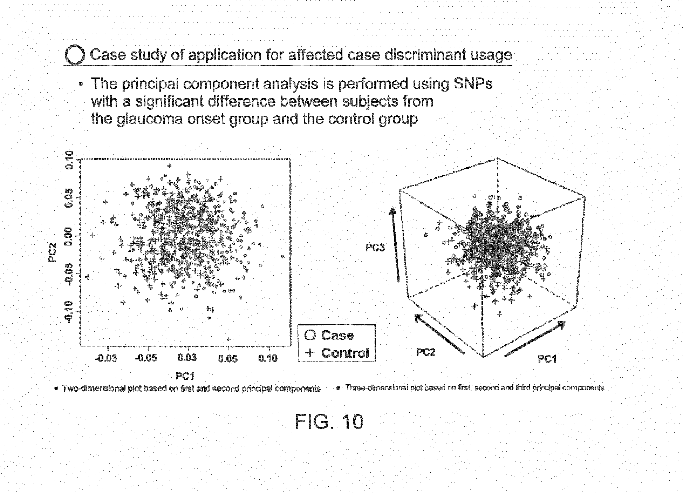

Fig. 10 is visual data describing an example of genotype data analysis by a principal component analysis used in the physiological condition discriminating apparatus of the present embodiment. A case study of an application of the principal component analysis for an onset case discriminant usage is shown in the figure. In other words, it shows a two-dimensional scatter diagram and a three-dimensional distribution diagram wherein principal component analysis was performed using SNPs with a significant difference between subjects from the glaucoma onset group and the non-onset group. In the figure, the analysis result is indicated as "o" for the onset group and "+" for the non-onset group.

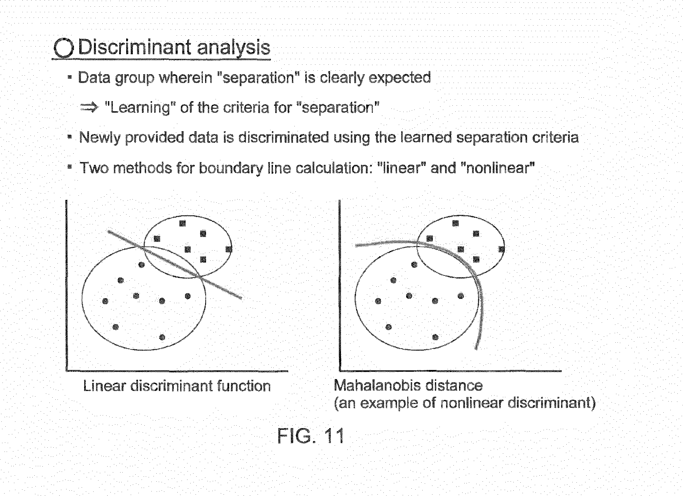

Fig. 11 shows a schematic diagram describing the principles of the discriminant analysis used in the physiological condition discriminating apparatus of the present embodiment. The discriminant analysis includes an analysis method wherein a standard for grouping is learned in advance and newly provided data is discriminated using the learned standard. There are two methods to calculate a boundary in a discriminant analysis, a linear discriminant and a non-linear discriminant (function using Mahalanobis distance and/or the like). Among many varieties used in a function for discriminant analysis (analysis engine) the following may be preferably used: "Ida" and "qda" that are built-in features of "MASS" written in "R"; and "Mahalanobis" that is a built-in feature of "stats" library.

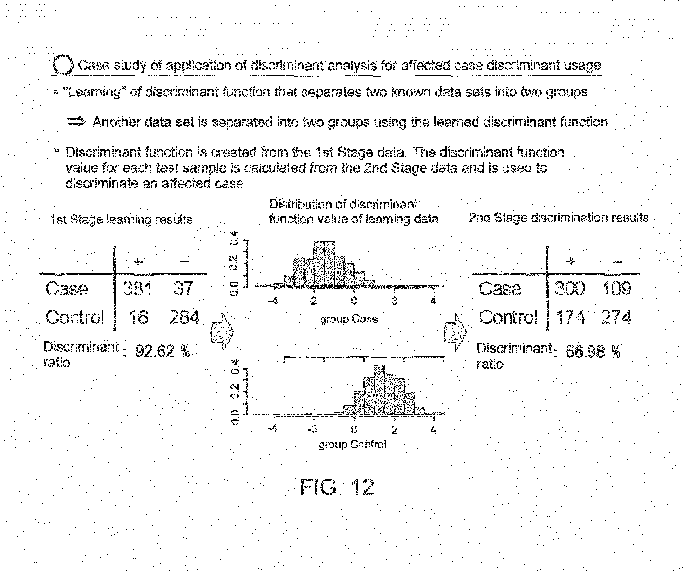

Fig. 12 shows visual data describing an analysis case of genotype data by the discriminant analysis used in a physiological condition discriminating apparatus of the present embodiment. A case study of an application of the discriminant analysis for an onset case discriminant usage is shown in the figure. For each sample of a control group and glaucoma onset group, measurement results are prepared by Affy 500k as a first stage, and measurement results are prepared by iSelect as a second stage. The first stage data is used as "learning data" in the creation of discriminant function, and the second stage data is used as "test data" for confirmation in the calculation of a discriminant function value for each sample, with which an affected case is discriminated. By conducting a discriminant analysis in such a manner, it is possible to create a discriminant function having a discriminant ratio of 92% (Case: onset group, and Control: control group) within data of the first stage, and to discriminate an affected case using second stage data with a discriminant ratio of 67% (Case: onset group, and Control: control group).

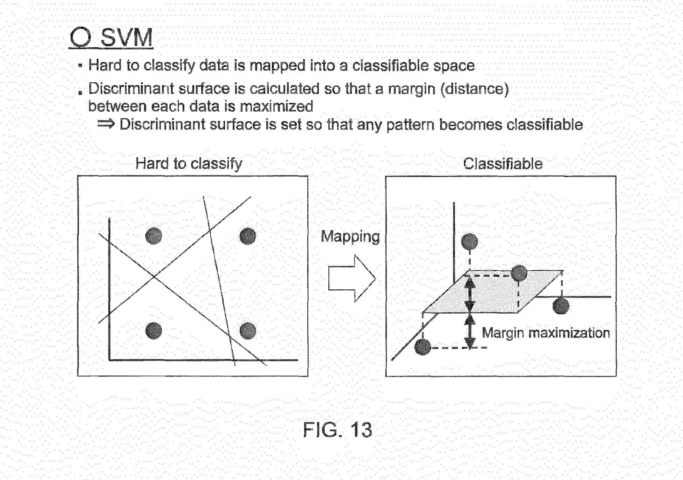

Fig. 13 shows a schematic diagram describing the principles of SVM used in the physiological condition discriminating apparatus of the present embodiment. SVM refers to an analysis method for calculating a discriminant surface that maximizes a margin (distance) between each data by mapping hard to classify data into a classifiable space via a kernel function. Using SVM, any data with any pattern can be accommodated by setting the appropriate kernel function to use. Among many varieties used in a function for SVM (analysis engine), the following may be preferably used: a method in which "ksvm" that is a built-in feature of "kernlab" written in "R" is used in combination with a kernel function such as 'rbfdot', 'polydot', 'vanilladot', 'tanhdot', 'laplacedot', 'besseldot', 'annovadot', and 'splinedot'; a method in which "svm" that is a built-in feature of "e1071" library is used in combination with a kernel function such as 'liner', 'polynomial ', 'radial'; and ' SVM ligh' and 'LIBSVB' libraries that can be used in the C language.

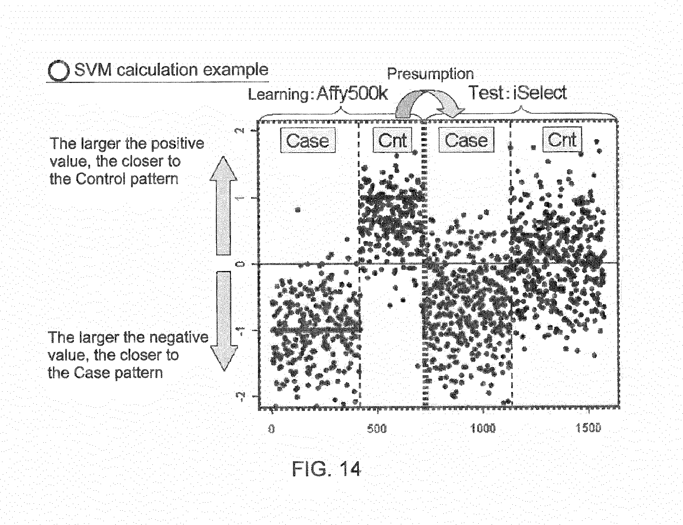

Fig. 14 shows visual data describing an analysis case of genotype data by SVM used in the physiological condition discriminating apparatus of the present embodiment. An example of a calculation by SVM is shown in this figure. Specifically, an assumption is made using a measurement result of iSelect as a test sample by learning the measurement result of the Affy 500k. Because SVM conducted learning so that the score of each sample approached -1 for the Case group and +1 for the Control group, the larger the positive value, the closer to the Control pattern and the larger the negative value, the closer to the case pattern. Using SVM, hard to classify data can be converted with the first stage data, and the utmost classifiable discriminant interface can be learned. Thereafter, it is possible to discriminate the positive and negative groups by scoring the distance from the discriminant interface with the second stage data.

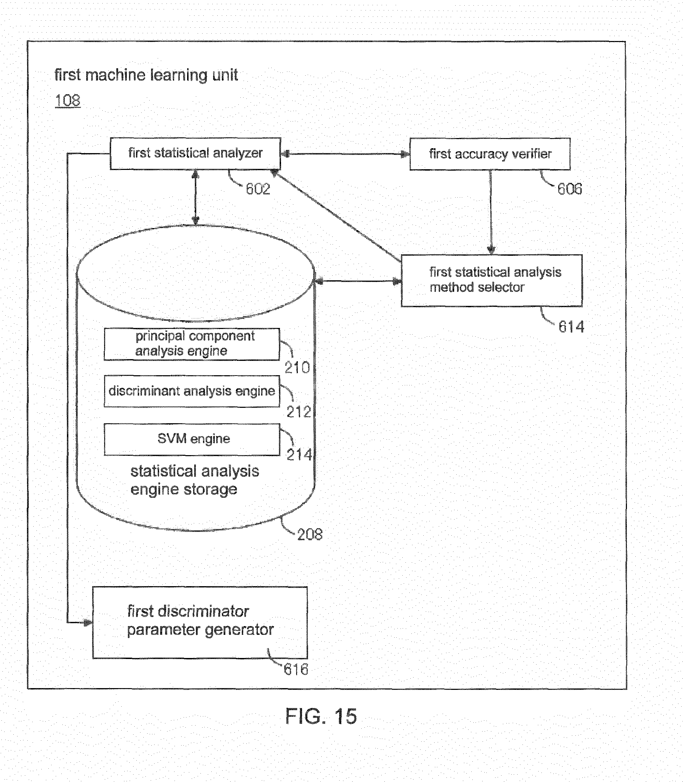

Fig. 15 is a functional block diagram that describes a configuration of a first machine learning unit 108 of a physiological condition discriminating apparatus 1000 of the present embodiment. The first machine learning unit 108 comprises a first statistical analyzer 602 that conducts at least one statistical analysis selected from a group consisting of a principal component analysis, a discriminant analysis, an SVM, a factor analysis, a cluster analysis, a multiple regression analysis, a decision tree, Naïve Bayes classifier, an artificial neural network, a Markov chain Monte Carlo method, a Gibbs sampling, and a SOM. Among those, it is preferable that the first statistical analyzer 602 conducts at least one statistical analysis method selected from the group consisting of the principal component analysis, discriminant analysis, and SVM even. The first machine learning unit 108 also comprises a statistical analysis engine storage 208 that stores various types of statistical analysis engines such as a principal component analysis engine 210 , a discriminant analysis engine 212, a SVM engine 214, and other engines (engines for analysis such as a factor analysis, a cluster analysis, a multiple regression analysis, a decision tree, a Naïve Bayes classifier, an artificial neural network, a Markov chain Monte Carlo method, a Gibbs sampling, and the SOM) for the above-described statistical analysis. The first statistical analyzer 602 conducts the SVM 100 times per 100 resample data. The number of different types of statistical analysis methods is not limited to a single method, and thus 2, 3, 4, 5, 6, 7, 8, 9, 10, 11, or even all 12 methods may be used. The number of different types of statistical analysis methods may be within the range of the two exemplified numerical values.

The first machine learning unit 108 also comprises a first accuracy verifier 606 that verifies a discrimination result of test data based on a SVM learning result of 100 batches for example. The test sample data may be obtained from test sample extractor 906 that is provided in resampler 106. By providing the first accuracy verifier 606, it is possible to determine which one of the following analysis engines can give the most accurate discrimination results: the principal component analysis engine 210 , the discriminant analysis engine 212, the SVM engine 214, and other engines (engines for performing analysis such as the factor analysis, the cluster analysis, the multiple regression analysis, the decision tree, the Naïve Bayes classifier, the artificial neural network, the Markov chain Monte Carlo method, the Gibbs sampling, and SOM) for the above-described statistical analysis.

The first machine learning unit 108 also comprises a first statistical analysis method selector 614. Based on the verification results by the first accuracy verifier 606, the first statistical analysis method selector 614 is configured to employ at least one statistical analysis method with the highest discrimination accuracy from the group consisting of the principal component analysis engine 210, the discriminant analysis engine 212, the SVM engine 214, and other engines (engines for performing analysis such as the factor analysis, the cluster analysis, the multiple regression analysis, the decision tree, the Naïve Bayes classifier, the artificial neural network, the Markov chain Monte Carlo method, the Gibbs sampling, and the SOM). The number of different types of statistical analysis methods is not limited to a single method, and 2, 3, 4, 5, 6, 7, 8, 9, 10, 11, or even all 12 methods may be used. The number of different types of statistical analysis methods may be within the range of the two exemplified numerical values.

The first machine learning unit 108 also comprises a first discriminator parameter generator 616, which is a discriminator using the SVM learning results of 100 batches, and/or the like. The first discriminator parameter generator 616 generates the first discriminator that numerically formulates the statistical analysis method with the maximum degree of discrimination accuracy selected by the first statistical analysis method selector 614 from the various statistical methods conducted by the first statistical analyzer 602. Prulal first discriminators thus obtained for prulal each subdata sets are sent to the below-described subject data analyzer 112 and utilized for a subject data analysis.

### Contiguous Data

The contiguous data of the present embodiment is data relating the blood cytokine concentration of an individual, as described hereafter. In other words, the result of a blood cytokine concentration measurement with CBA is used as the contiguous data. In other words, the measurement principle of the blood cytokine concentration measurement is as follows.

In CBA, it is possible to perform a simultaneous multi-item measurement of blood cytokine by using prulal beads having a capture antibody that specifically corresponds to each soluble protein of target cytokine and/or the like coated on a surface thereof, and having different fluorescent intensities for each capture antibody on the beads. Specifically, 1) a plasma sample is obtained by centrifugation of blood collected from the sample; 2) the plasma sample is reacted with a captured antibody on the bead surface; 3) each detection antibody to be labeled is reacted with phycoerythrin pigment (PE); and 4) using a flow cytometer, a type of antigen is determined by the fluorescent intensity of the beads, and an amount of each antigen is determined by the fluorescent intensity of PE labeled detection antibody.

In other words, such a measurement is possible by labeling the beads with two pigments at various ratios and determining the position of the beads. As a method other than CBA, it is possible to accurately, efficiently and very rapidly obtain the contiguous data necessary in analysis by an antibody chip that mounts an antibody that specifically binds to cytokine, by obtaining data derived from an analysis result of blood of an individual, and by making use of this as the contiguous data. It is also possible to accurately, efficiently, and very rapidly obtain the contiguous data necessary in analysis, by an antibody chip having an antibody array that specifically binds to cytokine, by obtaining data derived from an analysis result of blood of an individual, and by making use of this as the contiguous data.

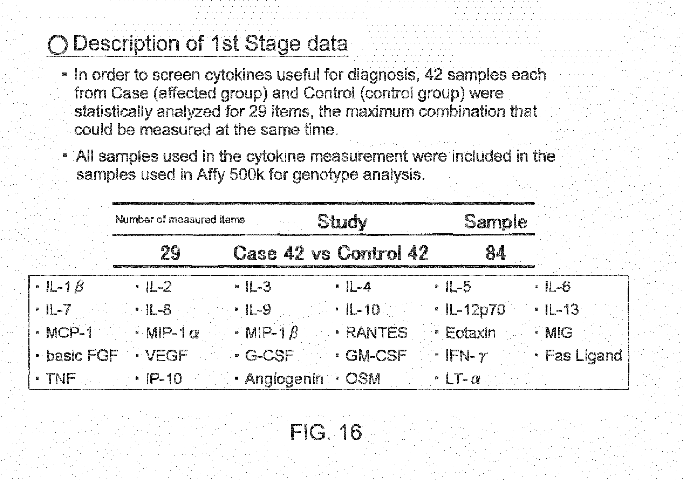

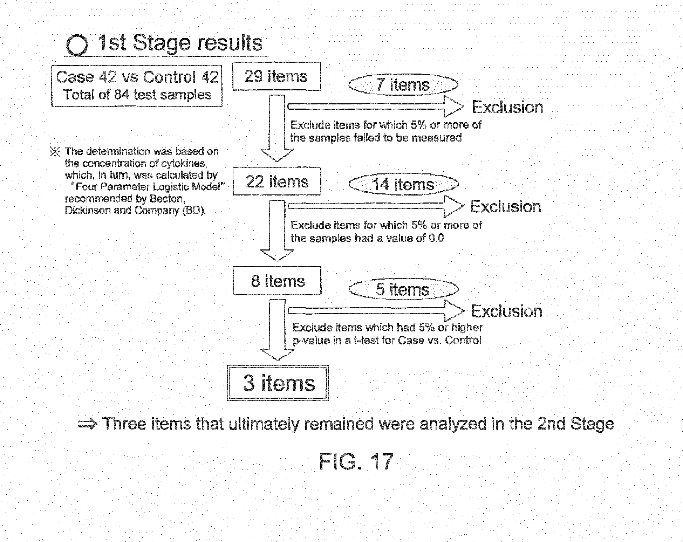

Figs. 16 and 17 show a schematic diagram for describing cytokine data that is used in the physiological condition discriminating apparatus of the present embodiment. The figure shows the sample information used for obtaining the cytokine data. Forty two samples were prepared as the glaucoma onset group and 42 samples were prepared as the control group for the obtaining cytokine data.

The following 29 types of blood cytokine concentrations were measured in blood collected from these subjects. A blood concentration was measured for at least one type of cytokine selected from the group consisting of IL-1β, IL-2, IL-3, IL-4, IL-5, IL-6, IL-7, IL-8, IL-9, IL-10, IL-12P70, IL-13, MCP-1(CCL2), MIP-1α(CCL3), MIP-1β(CCL4), RANTES(CCL5), Eotaxin(CCL11), MIG(CXCL9), basic-FGF, VEGF, G-CSF, GM-CSF, IFN-γ, Fas Ligand, TNF, IP-10, angiogenin, OSM, and LT-α. Specifically, the concentrations of 29 plasma cytokine items were measured using CBA as the first stage. The types of blood cytokines are not limited to a single type. Accordingly, 2, 3, 4, 5, 6, 7, 8, 9, 10, 11, 12, 13, 14, 15, 16, 17, 18, 19, 20, 21, 22, 23, 24, 25, 26, 27, or even all 29 cytokines may be used. The number of the types of blood cytokines may be within the range of the two exemplified numerical values.

As a result of the concentration measurement of the first stage, 7 items for which 5% or more of the samples failed to be measured were excluded. Next, 14 items for which 5% or more of the samples had a measurement value of 0.0 were excluded. Five items that had 5% or higher p-value in a t-test for Case vs. Control were excluded, and ultimately narrowed down to three items.

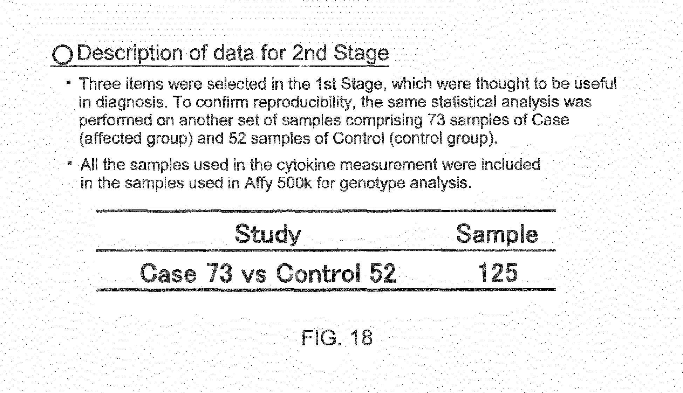

Fig. 18 shows a schematic diagram for describing the cytokine data used in the physiological condition discriminating apparatus of the present embodiment. For three items that were thought to be useful in diagnosis as a result of the first stage, statistical analysis was performed on 73 Cases (onset group) and 52 Controls (control group) from a different sample group in order to confirm reproducibility.

All the samples used in the measurement of these cytokines are included in the samples used for the Affy 500k genotype.

The cytokine data thus obtained undergoes a unique data standardization based on control group data in the cytokine data standardizer 812 of the learning data set acquiring unit 102 that is already described with Fig. 7. The cytokine used in the analysis is then selected. In the below-described second machine learning unit 110, the standardized cytokine is subjected to various statistical analyses similar to the analyses used for genotype data (e.g., the principal component analysis, the discriminant analysis, the SVM (support vector machine), the discriminant analysis, the factor analysis, the cluster analysis, the multiple regression analysis, the decision tree, the Naïve Bayes classifier, the artificial neural network, the Markov chain Monte Carlo method, the Gibbs sampling, and SOM). For more details, refer to the description below.

### Second machine learning unit

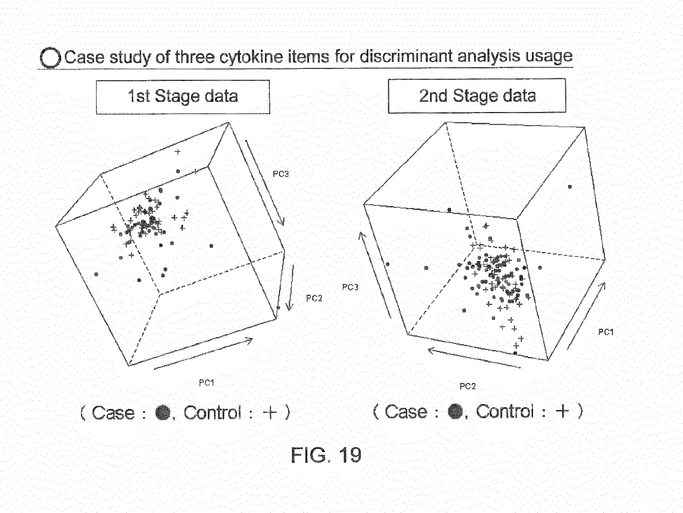

Fig. 19 shows visual data that describes an analysis case of the cytokine data by the principal component analysis used in the physiological condition discriminating apparatus of the present embodiment. In the figure, the blood cytokine concentrations indicated in Fig. 19 are measured, and the principal component analysis is performed together with a classification result of an attribute of a physiological condition regarding the presence and progression of glaucoma according to a diagnosis matching that confirmed by a medical doctor. This figure was created based on the first and second stage sample data, onset group vs. control group, and three cytokine items. Because the data is for three cytokine items, a 3-D plot was created in order to visualize all principal components, PC1-PC3. From the figure, it can be seen that when conducting an analysis on the three types of primary components, PC1, PC2, and PC3, the control group data, in general, is relatively clustered together, while the data of the onset group is scattered. This indicates high accuracy for discrimination of the attribute of a physiological condition of the onset of glaucoma (onset/healthy).

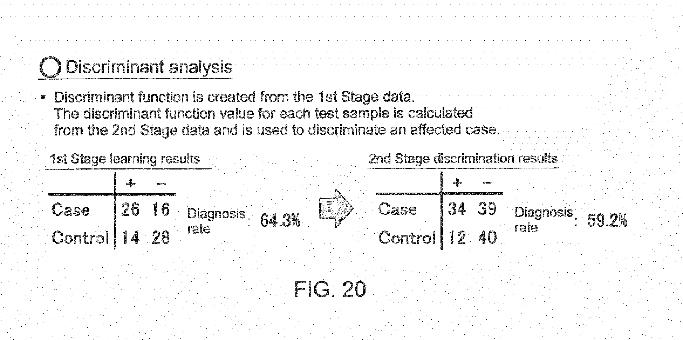

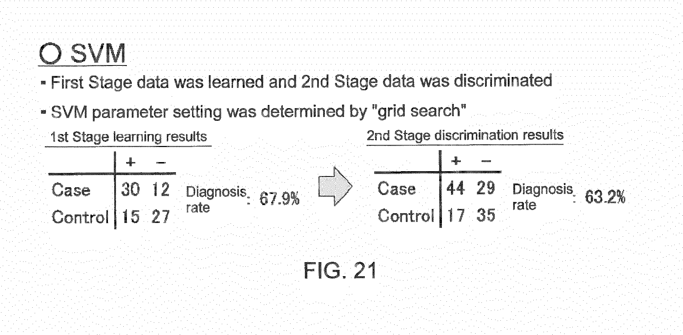

Figs. 20 and 21 show visual data describing cytokine data analysis case by discriminant analysis or SVM that is used in the physiological condition discriminating apparatus of the present embodiment. An estimated result of test data with a pattern extracted from discriminant analysis or SVM learning data is shown in Figs. 20 and 21. Specifically, for the discriminant analysis, a discriminant function is created from the first stage data, and a discriminant function value for each sample is calculated from the second stage data, and an affected case is discriminated by that value. For SVM, the first stage data is learned, and the SVM parameter that discriminates the second stage data is determined by a "grid search".

Specifically, for the discriminant analysis, a discriminant function is created from the first stage data, and a discriminant function value for each sample is calculated from the second stage data, and an affected case is discriminated by that value. For SVM, the first stage data is learned, and the second stage data is discriminated. A SVM parameter setting is determined with a grid search. Any of the principal component analysis, the discriminant analysis, the SVM, and other engines (engines for performing analysis such as the factor analysis, the cluster analysis, the multiple regression analysis, the decision tree, the Naïve Bayes classifier, the artificial neural network, the Markov chain Monte Carlo method, the Gibbs sampling, and SOM) may be suitably used when machine learning contiguous data such as the blood cytokine concentration in a physiological condition discriminating apparatus of the present embodiment.

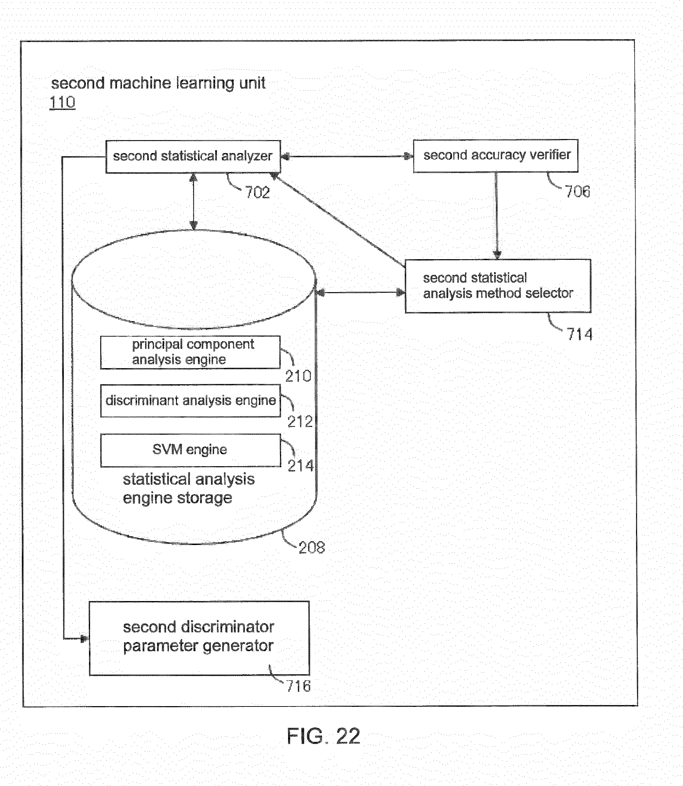

Fig. 22 shows a functional block diagram that describes a configuration of the second machine learning unit 110 of the physiological condition discriminating apparatus 1000 of the present embodiment. The second machine learning unit 110 comprises a second statistical analyzer 702 that conducts at least one statistical analysis method selected from the principal component analysis, the discriminant analysis, the SVM, the factor analysis, the cluster analysis, the multiple regression analysis, the decision tree, the Naïve Bayes classifier, the artificial neural network, the Markov chain Monte Carlo method, the Gibbs sampling, and the SOM. The second machine learning unit 110 also comprises the statistical analysis engine storage 208 that stores various types of statistical analysis engines such as a principal component analysis engine 210, the discriminant analysis engine 212, the SVM engine 214, and other engines (engine for analysis such as a factor analysis, a cluster analysis, a multiple regression analysis, a decision tree, a Naïve Bayes classifier, an artificial neural network, a Markov chain Monte Carlo method, a Gibbs sampling, and the SOM). The second statistical analyzer 702 conducts a machine learning of a pattern of an attribute of a physiological condition and contiguous data included in the prulal subdata sets by reading out any of the analysis engines such as the principal component analysis engine 708, the discriminant analysis engine 710, the SVM engine 712, and other engines (engines for performing analysis such as a factor analysis, a cluster analysis, a multiple regression analysis, a decision tree, a Naïve Bayes classifier, an artificial neural network, a Markov chain Monte Carlo method, a Gibbs sampling, and the SOM) from the statistical analysis engine storage 208. The number of different types of statistical analysis methods is not limited to a single method, and thus, 2, 3, 4, 5, 6, 7, 8, 9, 10, 11, or even all 12 methods may be used. The number of different types of statistical analysis methods may be within the range of the two exemplified numerical values.

The second machine learning unit 110 also comprises a second accuracy verifier 706 that verifies the discrimination accuracy of the sample result obtained by pattern analyzing the test sample data that is randomly extracted from the learning data set using the second discriminator. The test sample data may be obtained from the test sample extractor 906 that is provided in the resampler 106. By providing the second accuracy verifier 706, it is possible to determine which one of the following analysis engines can give the most accurate discrimination results: the principal component analysis engine 210, the discriminant analysis engine 212, the SVM engine 214, and other engines (engines for performing analysis such as the factor analysis, the cluster analysis, the multiple regression analysis, the decision tree, the Naïve Bayes classifier, the artificial neural network, the Markov chain Monte Carlo method, the Gibbs sampling, and the SOM).

The second machine learning unit 110 also comprises a second statistical analysis method selector 714. Based on the verification results by the second accuracy verifier 706, the second statistical analysis method selector 714 is configured to employ at least one statistical analysis method with the highest discrimination accuracy selected from the group consisting of the principal component analysis engine 210, the discriminant analysis engine 212, the SVM engine 214, and other engines (engines for performing analysis such as the factor analysis, the cluster analysis, the multiple regression analysis, the decision tree, the Naïve Bayes classifier, the artificial neural network, the Markov chain Monte Carlo method, the Gibbs sampling, and the SOM). The number of different types of statistical analysis methods is not limited to a single method, and 2, 3, 4, 5, 6, 7, 8, 9, 10, 11, or even all 12 methods may be used. The number of different types of statistical analysis methods may be within the range of the two exemplified numerical values.

The second machine learning unit 110 also comprises a second discriminator parameter generator 716. The second discriminator parameter generator 716 generates the second discriminator that numerically formulates the statistical analysis method with the maximum degree of discrimination accuracy selected by the first statistical analysis method selector 714 from the various statistical methods conducted by the second statistical analyzer 702. Prulal second discriminators thus obtained for prulal each subdata sets are sent to the below-described subject data analyzer 112 and utilized for a subject data analysis.

### Subject data acquiring unit

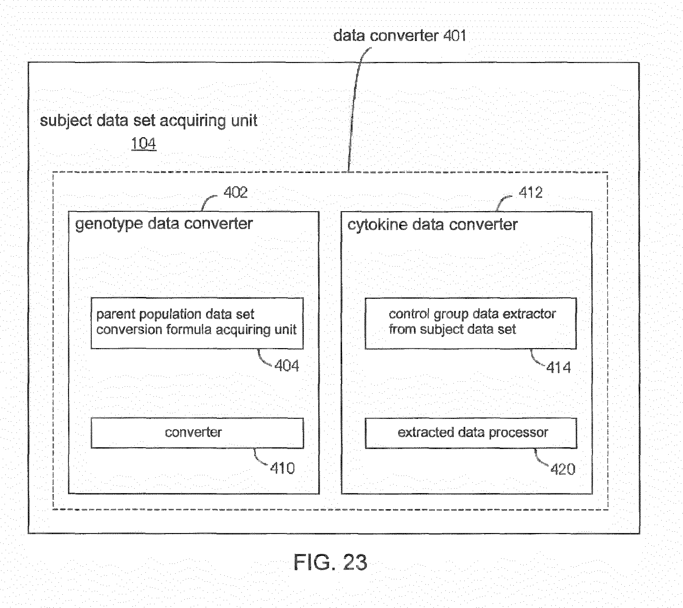

Fig. 23 shows a functional block diagram describing a configuration of the subject data set acquiring unit 104 of the physiological condition discriminating apparatus 1000 of the present embodiment. The subject data set acquiring unit 104 is configured to acquire the subject data on a subject individual, including a combination of the discrete data relating to a gene polymorphism of an individual and the contiguous data relating to blood cytokine concentrations of an individual.

The subject data set acquiring unit 104 comprises a data converter 401 that digitizes and/or normalizes the subject data with a method similar to that used for the learning data set. The data converter 401 comprises a genotype data converter 402 that digitizes and/or normalizes the genotype data included in the obtained subject data. The genotype data converter 402 comprises a learning data set conversion formula acquiring unit 404 that acquires a digitization and/or normalization method in the learning data set from the learning data set acquiring unit 102. The genotype data converter 402 also comprises a converter 410 that digitizes and/or normalizes the genotype data included in the subject data using the digitization and/or normalization method of the learning data set thus obtained. In Fig. 7, the allele frequency calculator 808 in the learning data set acquiring unit 102 is configured to acquire the data (information on average value and allele frequency in the learning data set for each SNP) needed by the learning data set conversion formula acquiring unit 404, for normalizing according to the distribution of the learning data set.

The data converter 401 also comprises a cytokine data converter 412 that digitizes and/or normalizes the cytokine data included in the obtained subject data. With regard to a contiguous value of cytokine and/or the like, because it is possible to handle each analysis similarly to a learning data set value when normalized to a standard normal distribution value, it is not necessary to acquire some type of data or conversion formula from the learning data set. Due to the nature of CBA, not only a single sample, but at least multiple sample units (basically several tens of samples) are measured simultaneously. Accordingly, data of the control group should be obtained in each measurement, and functions as the basis for at least several samples. Normalization is possible using the data of the control group without the learning data set. There is no need for acquiring anything from the learning data set in the cytokine data converter 412. Instead, the cytokine data converter 412 requires a control data extractor 414 that extracts control group data within the subject data set as well as an extracted data processor 420 that calculates an average value and a standard deviation.

An extracted data storage (not shown in the figure) may be provided that calculates the standard deviation and the average value by extracting only the control group from the subject data set (prulal individuals) once, and locally and temporarily stores them. In this manner, it is possible to normalize for a certain individual inputted to the cytokine data converter 412 by loading a pre-stored average value and standard deviation. This eliminates a need for repeatedly calculating standard deviation and average value while normalizing an entire subject dataset (prulal individuals).

This system can be expanded even further to include all subject data sets that were used in the past (anonymous from an ethical perspective). According to the range of the input value, a standard deviation and an average can be loaded which are empirically calculated from the past subject data sets. In such a case, a normalization parameter seta can be used for an inputted cytokine A value below 50, and a normalization parameter setβ can be used for a value between 50-100.

### Subject data analyzer

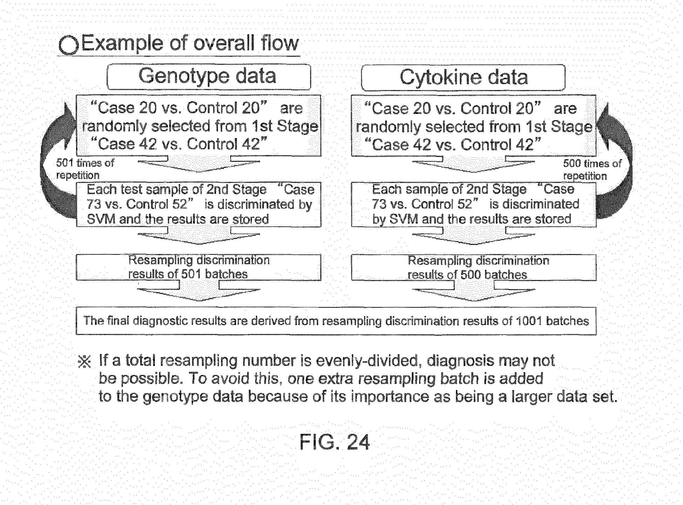

Fig. 24 shows a schematic diagram describing a function of an integration discriminator 114 of the physiological condition discriminating apparatus 1000 of the present embodiment. As previously described, it is difficult to simply sum up two types of data that have different numerical characteristics such as the genotype data which is a discrete value, and the cytokine data which is a contiguous value. In the present embodiment, the attribute of the physiological condition is determined by integrating each discrimination result instead of the numerical value. Specifically, the two analyses are integrated based on a method of bagging. In other words, a process is performed based on a method of bagging using the genotype data as step 1, a process is performed based on a method of bagging using the cytokine data as step 2, the results of each of steps 1 and 2 are integrated as step 3, and a final determination was made using a majority decision. In practice, the test was conducted under the following conditions.

Resampling and learning / estimating was conducted on the genotype 501 times and on the cytokine 500 times, and the results were discriminated by using a majority decision from two learning results. As the learning data, a random selection was made from 42 healthy individuals and 42 samples of first stage glaucoma (population having Affy 500k genotype data, which is the same as a first stage of the cytokine) to obtain the equal number for each group (20 samples each). As the test data, 52 healthy individuals and 73 samples of second stage glaucoma (population having Affy 500k genotype data, which is the same as a second stage of the cytokine) were utilized.

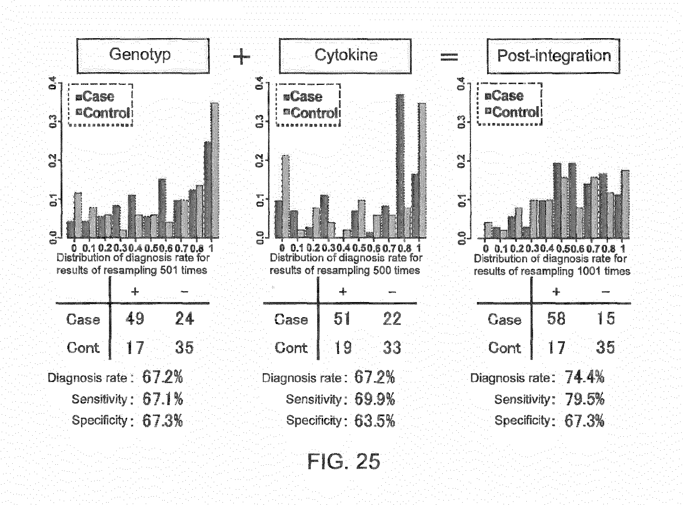

Fig. 25 shows visual data describing the integrated determining unit 114 of the physiological condition discriminating apparatus 1000 of the present embodiment. As indicated in the figure, resampling and learning / estimating was conducted on the genotype 501 times and on the cytokine 500 times, and the results were discriminated by using a majority decision from two learning results, as described above. In other words, from the 1001 times of resampling discrimination, a majority discrimination result was determined as the final attribute of a physiological condition. As a result, while the diagnosis rate of each of the 501 batches of the genotype and the 500 batches of the cytokine was 67.2%, the diagnosis rate after integration was clearly improved at 74.4%.

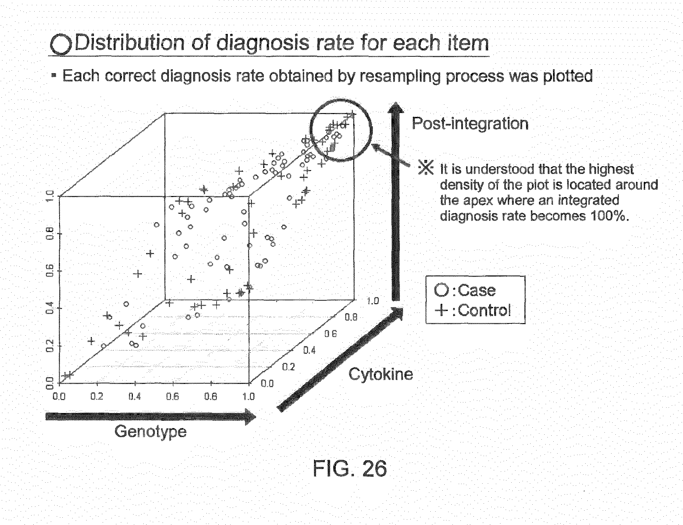

Fig. 26 is visual data describing the integration results of the genotype data and the cytokine data using the integrated determining unit 114 of the physiological condition discriminating apparatus 1000 of the present embodiment. As indicated in the figure, when plotting each ratio of correctly discriminated result by the resampling process, the highest density of the plots is seen around the apex where an integrated diagnosis rate is 100%. Accordingly, the discrimination accuracy is clearly improved by integrating the discrimination results from 501 resamplings of the genotype and 500 resampling of the cytokine.

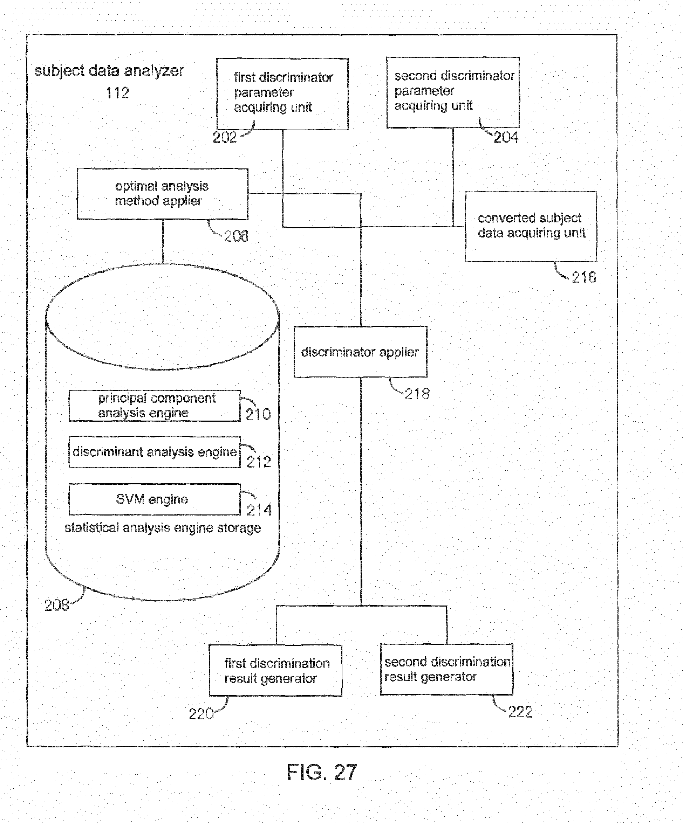

Fig. 27 shows a functional block diagram that describes a configuration of the subject data analyzer 112 of the physiological condition discriminating apparatus 1000 of the present embodiment. The subject data analyzer 112 comprises the first discriminator parameter acquiring unit 212 that acquires the first discriminator parameter from the first machine learning unit 108. The subject data analyzer 112 comprises a second discriminator parameter acquiring unit 204 that acquires the second discriminator parameter from the second machine learning unit 110. As prulal first discriminators and second discriminators, an optimal analysis method applier 206 is provided which uses statistical analysis method with the maximum discrimination accuracy selected from the group consisting of the principal component analysis, the discriminant analysis, the SVM, the factor analysis, the cluster analysis, the multiple regression analysis, the decision tree, the Naïve Bayes classifier, the artificial neural network, the Markov chain Monte Carlo method, the Gibbs sampling, and the SOM. The number of different types of statistical analysis methods is not limited to a single method, and thus, 2, 3, 4, 5, 6, 7, 8, 9, 10, 11, or even all the 12 methods may be used. The number of different types of statistical analysis methods may be within the range of the two exemplified numerical values.

The subject data analyzer 112 comprises a statistical analysis engine storage 208 that stores the principal component analysis engine 210, the discriminant analysis engine 212, the SVM engine 214, and other engines (engines for performing analysis such as the factor analysis, the cluster analysis, the multiple regression analysis, the decision tree, the Naïve Bayes classifier, the artificial neural network, the Markov chain Monte Carlo method, the Gibbs sampling, and the SOM). The optimal analysis method applier 206 transfers any analysis engine of the principal component analysis engine 210, the discriminant analysis engine 212, the SVM engine 214, and other engines (engines for performing analysis such as the factor analysis, the cluster analysis, the multiple regression analysis, the decision tree, the Naïve Bayes classifier, the artificial neural network, the Markov chain Monte Carlo method, the Gibbs sampling, and the SOM) necessary for analysis using an obtained first discriminator and second discriminator, to a discriminator applier 218 by reading out from the statistical engine storage 208.

The subject data analyzer 112 comprises a converted subject data acquiring unit 216 that digitizes or normalizes by a method that is the same as the learning data set obtained by the subject data set acquiring unit 104. The subject data analyzer 112 also comprises a discriminator applier 218, which generates the first discrimination result and the second discrimination result of an attribute of a physiological condition of the subject individual by pattern analyzing the subject using at least one of prulal first discriminators and second discriminators that are different from each other.

Accordingly, a first discrimination result based on the genotype data and a second discrimination result based on the cytokine data are obtained for any of the prulal setup subdata sets in the subject data analyzer 112. The first discrimination result based on the genotype data and the second discrimination result based on the cytokine data of these plurality subdata sets are each compiled into the two types of data sets by the first discrimination result generator 220 and the second discrimination result generator 222 and sent to the integrated determining unit 114 described below.

### Integrated determining unit

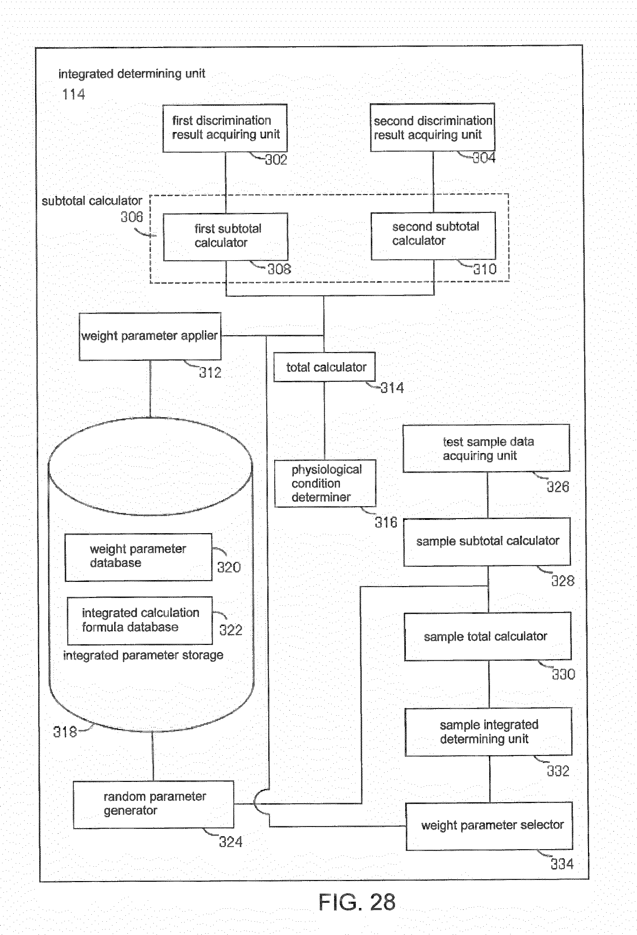

Fig. 28 shows a functional block diagram describing a configuration of the integrated determining unit 114 of the physiological condition discriminating apparatus 1000 of the present embodiment. As shown in the figure, the integrated determining unit 114 comprises a first discrimination result acquiring unit 302 that acquires the first discrimination result based on the genotype data from the subject data analyzer 112. The integrated determining unit 114 also comprises a second discrimination result acquiring unit 304 that acquires the second discrimination result based on the genotype data from the subject data analyzer 112.

The integrated determining unit 114 comprises a subtotal calculator 306 that provides a subtotal of each number in which the subject data in the first discrimination result and the second discrimination result are determined as a specific attribute of a physiological condition. The subtotal calculator 306 comprises a first subtotal calculator 308 that calculates the subtotal of the first discrimination result based on the genotype data. The subtotal calculator 306 also comprises a second subtotal calculator 310 that calculates the subtotal of the second discrimination result based on the cytokine data. The integrated determining unit 114 also comprises a total calculator 314 that calculates the total of a subtotal result of the first discrimination result based on the genotype data and the second discrimination result based on the cytokine result for each attribute of a physiological condition.

The integrated determining unit 114 further comprises a weight parameter applier 312, which calculates a total weight of each weight of the subtotal results according to the predetermined parameter. The subtotal results are obtained from the first discrimination result based on the genotype data, and the second discrimination result based on the cytokine result. The integrated determining unit 114 also comprises an integrated parameter storage 318 that is connected to the weight parameter applier 312.

The integrated parameter storage 318 stores a weight parameter database 320 that stores a weight parameter that is thought to be optimal at the present time based on discrimination accuracy information such as the test result of the sample data or a past discrimination result. The integrated parameter storage 318 also stores an integrated calculation formula database 322 that stores an integration calculation formula for integrating a subtotal result of the first subtotal calculator 308 and the second subtotal calculator 310 using a weight parameter thereof.

The integrated determining unit 114 comprises a test sample data acquiring unit that acquires the obtained sample analysis result by processing the test sample data that is randomly extracted from the learning data set with subject data analyzer 112. The integrated determining unit 114 comprises a sample subtotal calculator 328, which obtains each of the subtotal results based on the genotype data and the subtotal results based on the cytokine data with regard to the sample analysis result thus obtained.

The integrated determining unit 114 also comprises a random parameter calculator 324 that randomly generates prulal weight parameters. The integrated determining unit 114 also comprises a sample total calculator 330, which calculates the total of each of the sample subtotals for each attribute of a physiological condition after the application of weighting by a random weight parameter thus generated. The integrated determining unit 114 also comprises a integrated determining unit 332, which integrally determines the attribute of a physiological condition that is most frequently discriminated as an attribute of a physiological condition of a sample individual by counting for each sample individual included in the test sample data in the sample total result. The integrated determining unit 114 also comprises a weight parameter selector 334, which employs the weight parameter with the maximum determination accuracy by adding up the determination accuracy of the integrated determination results of every sample individual for each weighed parameter.

Accordingly, it is possible to perform integrated determination with the total calculator 314 by applying a weight parameter that is thought to be the optimal parameter thereof after selecting a weight parameter that is thought to be optimal based on the discrimination result of the test sample obtained by using the test sample extractor 906 of the resampler 106 in the integrated determining unit 114. The attribute of a physiological condition with the highest discrimination frequency among the discrimination results thus obtained by total calculator 314 is determined as the final integrated determination result.

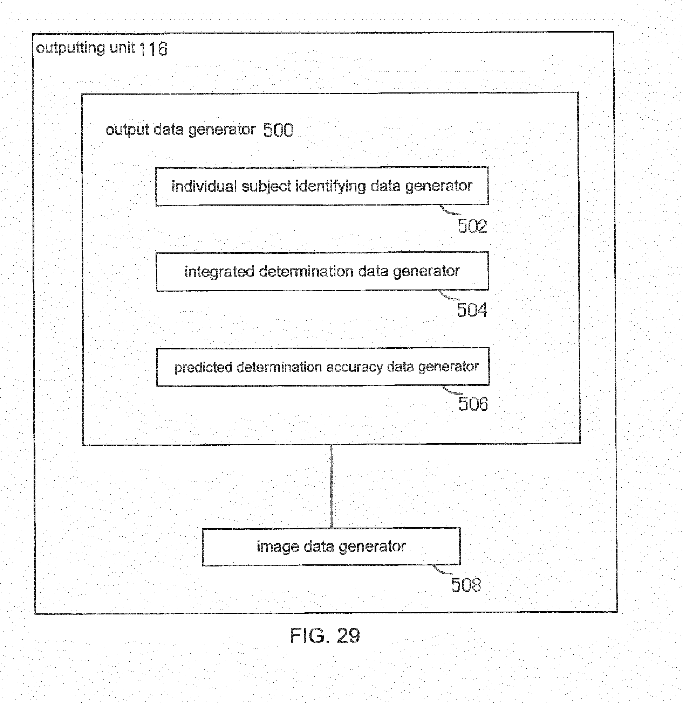

Fig. 29 is a functional block diagram describing a configuration of the outputting unit 116 of the physiological condition discriminating apparatus 1000 of the present embodiment. The outputting unit 116 comprises an output data generator 500 that generates the data set relating to the integrated determination result according to the integrated determining unit 114. The output data generator 500 comprises a data generator for identifying individual subject 502 for generating data that identifies the individual subject. The output data generator 500 also comprises an integrated determination data generator 504 for generating data that indicates the result of the integrated determination. The output data generator 500 comprises a predicted determination accuracy data generator 506 for generating data that indicates the predicted determination accuracy.

The outputting unit 116 comprises an image data generator 508 that generates image data indicating the contents of the data set relating to the generated integrated determination results of the output data generator 500. The image data generated by the image data generator 508 may be displayed on the image display 130, or may be printed with the printer 132, or may be written on the server 134, via the network 120 such as a LAN or Internet.

### Operation of physiological condition discriminating apparatus

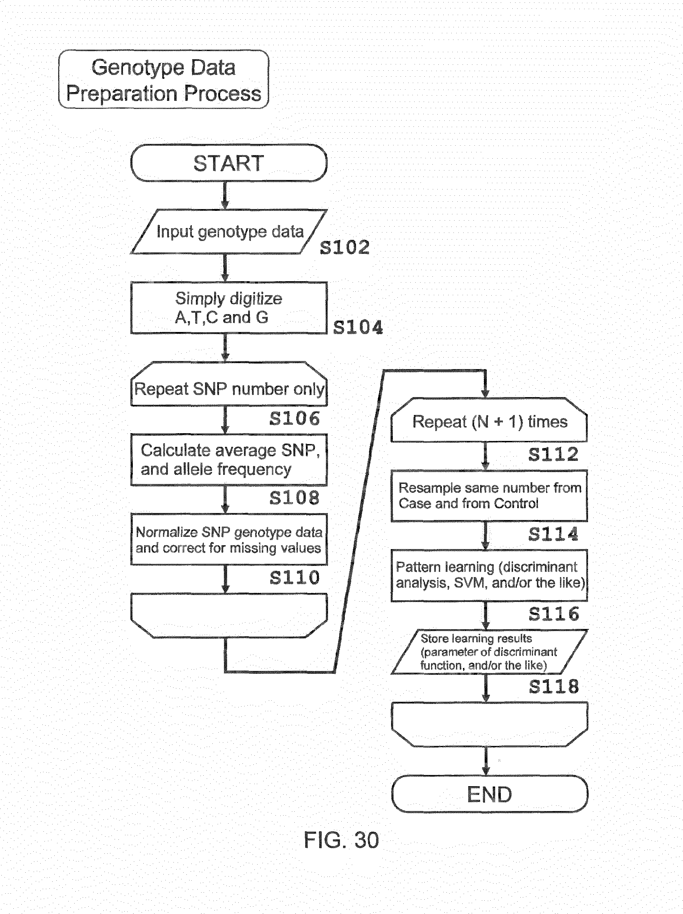

Fig. 30 shows a flowchart describing an analytic operation of the genotype data of the physiological condition discriminating apparatus 1000 of the present embodiment. As indicated in the figure, when the physiological condition discriminating apparatus 1000 starts a series of analytic operation of genotype data, at first, the input of the genotype data is accepted in the learning data set acquiring unit 102 (S102). Then, with regard to the genotype data that is thus input, A,T,C and G are simply digitized in a genotype data digitizer 802 of the learning data set acquiring unit 102 (S104).

On the genotype data that has been thus digitized, the average value of SNP and allele frequency are calculated in an allele frequency calculator 808 of the learning data set acquiring unit 102 (S108), and then a missing value is corrected by normalizing the SNP genotype data in a normalizer 810 in a similar manner (S110). The processes of S108 and S110 are repeated for the same number of times as the SNP number (S106).

Next, from the genotype data that has been thus normalized, the same number each of Case (glaucoma) and Control (healthy individual) are resampled in the resampler 106 (S114). On prulal subdata sets that has been thus resampled, a pattern learning (e.g., discriminant analysis, SVM, and others) is performed respectively in the first machine learning unit 108 (S116). The learning result that has been thus learned by pattern learning is then sent from the first machine learning unit 108 to the subject data analyzer 112 where it is temporarily stored (S118). The processes of S114, S116 and S118 are repeated N + 1 times (S112) before the completion of a series of operation.

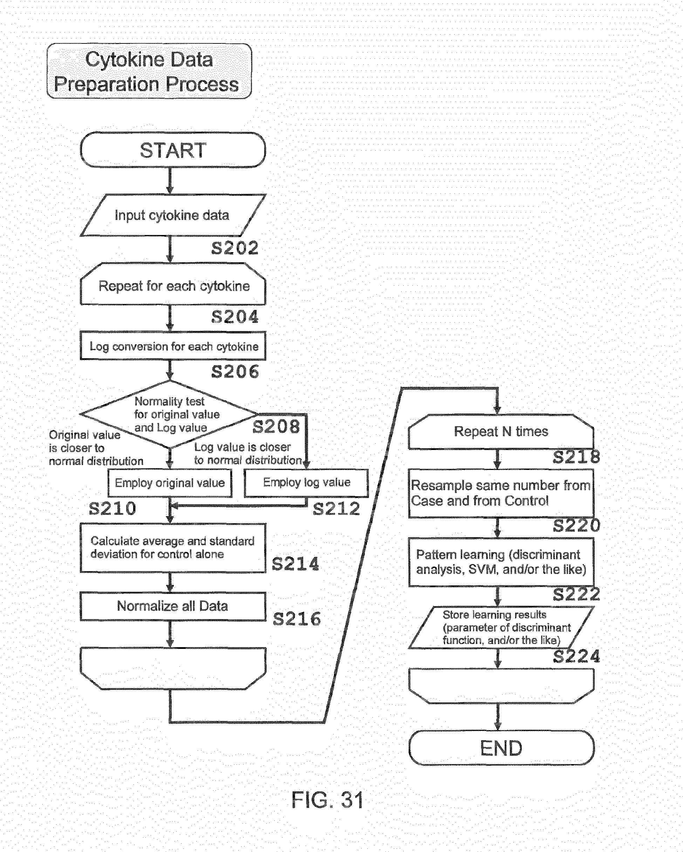

Fig. 31 shows a flowchart describing an analytic operation of the cytokine data of the physiological condition discriminating apparatus of the present embodiment. As indicated in this figure, when the physiological condition discriminating apparatus 1000 starts a series of analytic operation of cytokine data, at first, the input of the cytokine data is accepted in the learning data set acquiring unit 102 (S202). Then, the cytokine data that is thus input is converted into Log form in the Log converter 816 of the learning data set acquiring unit 102 (S206). In the Log converter, conversion may be performed by a common logarithm, conversion may be performed by a natural logarithm, or conversion may be performed by another base.

An original cytokine data value and a Log value thus obtained are tested about normality in a normality determiner 818 of the learning data set acquiring unit 102 (S208), and the original value is employed when the original value has a higher normality (S210), and the Log value is employed when the Log value has a higher normality (S212). The processes S206, S208, S210 and S212 are then repeated for each cytokine (S204), and then the control group data extractor 814 of the learning data set acquiring unit 102 calculates an average value and a standard deviation thereof by extracting the control group data from the parent population data (S214). The standardizer of the learning data set acquiring unit 102 normalizes (standardizes) all the data by using an average value and a standard thus obtained (S216).

Next, with regard to the cytokine data that is thus normalized (standardized), the same numbers are respectively resampled from the Case (glaucoma) and the Control (healthy individual) in the resampler 106 (S220). On the prulal subdata sets that are thus resampled, pattern learning (e.g., discriminant analysis and SVM) is respectively performed in the second machine learning unit 110 (S222). The learning result that is thus learned by pattern learning is sent from the second machine learning unit 110 to the subject data analyzer 112 and temporarily stored (S224). The processes of S220, S222 and S224 are then repeated for N times (S218) before the completion of a series of operations.

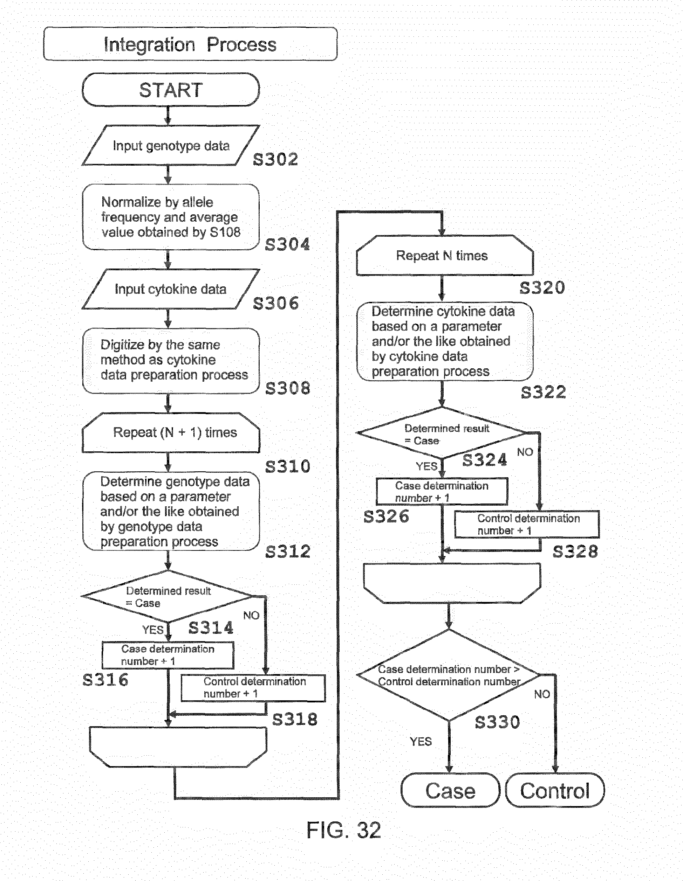

Fig. 32 shows a flowchart describing an analytic operation of subject data of the physiological condition discriminating apparatus of the present embodiment. As indicated in the figure, when the physiological condition discriminating apparatus 1000 starts a series of integrated determination operations, at first, an input of the genotype data is accepted in the subject data acquiring unit 104 (S302). Then, the genotype data that is thus input is normalized using the allele frequency and the average value obtained by S108 in a genotype data converter 402 of the subject data acquiring unit 104 (S304). In the calculation performed in S108, the learning data set characteristics are considered to be analogous to genome characteristics.

Next, an input of the cytokine data is accepted in the subject data acquiring unit 104 (S306). Afterwards, the cytokine data that is thus input is converted into numerical data by digitization or normalization method similarly to that of the learning data set in the cytokine data converter 412 of the subject data acquiring unit 104 (S308).

The genotype data of the subject data in the discriminator applier 218 of the subject data analyzer 112 is discriminated based on a parameter of prulal first discriminators and/or the like that correspond to the prulal subset data obtained in the learning process of the genotype data in the first machine learning unit 108 (S312). Each of the prulal first machine learning units determines whether the determination result is a Case (glaucoma) (S314). In a case where a determination result is a glaucoma determination, +1 point is awarded to a Case determination (S316), and in a case where the determination is a healthy individual determination, +1 point is awarded to a Control determination (S318). The processes of S312, S314, S316, and S318 are then repeated for N + 1 times (S310).

The cytokine data of the subject data in a discriminator applier 218 of the subject data analyzer 112 is discriminated based on a parameter of prulal second discriminators and/or the like that corresponds to the prulal subset data obtained in the learning process of the cytokine data in the second machine learning unit 108 (S322). Each of the prulal second machine learning units determines whether the determination result is a Case (glaucoma) (S324). In a case where a determination result is a glaucoma determination, +1 point is awarded to a Case determination (S326), and in a case where a determination is a healthy individual determination, +1 points is awarded to the Control determination (S328). The processes of S322, S324, S326, and S328 are then repeated for N times (S320).

The reason for repeating the genotype analysis N + 1 times and the cytokine analysis N times is as follows: if the weight of both processes is 1:1 and both process are repeated for N times, the final determination result could be N:N, which makes it impossible to discriminate between the Case and the Control. By using an odd number instead of an even number for the total processing time, a discrimination between the Case and the Control is always guaranteed. Accordingly, a decision to repeat the genotype analysis one more time was made rather than the cytokine analysis, since the former is considered to be more reliable.

Finally, the determination result of the genotype data and the determination result of the cytokine data are integrated by the integrated determining unit 114 in order to compare the Case determination frequency and the Control determination frequency (S330). The result is determined to be a Case (glaucoma), if the Case determination frequency is larger; and result is determined to be a Control (healthy individual), if the Control determination frequency is larger before the completion of a series of operations.

### Modified Embodiment

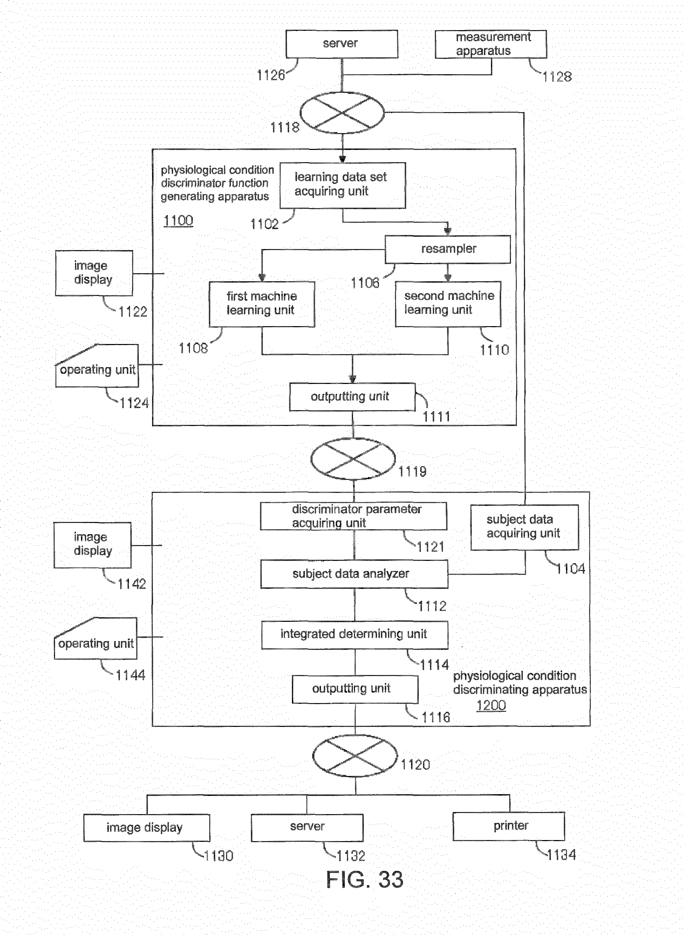

Fig. 33 shows a functional block diagram for describing a modification of the present embodiment. The physiological condition discriminator parameter generating apparatus 1100 according to the present embodiment is a apparatus for generating a discriminator using a discrimination method of a physiological condition that is described by the flowchart. The physiological condition discriminator parameter generating apparatus 1100 comprises a learning data set acquiring unit 1102 that acquires a learning data set, wherein the data set relates to a group of individuals consisting of prulal individuals used in the below-described machine learning, wherein the group of individuals is obtained from a parent population consisting of individuals belonging to the same species as the subject individual, and wherein the data set includes a combination of an attribute of a physiological condition of the individual, discrete data relating to a genomic base sequence of the individual, and contiguous data relating to an amount of a specific substance in the individual organism.

The physiological condition discriminator parameter generating apparatus 1100 comprises a resampler 1106 that extracts a subdata set from the above-described learning data set, wherein the subdata set relates to prulal subgroups that differ from each other, the subdata set constituting a part of the group of individuals. The resampler 1106 includes a combination of the attribute of a physiological condition of each individual included in the subgroups of individuals, the discrete data relating to a genomic base sequence of the each individual, and the contiguous data relating to an amount of a specific substance in each of the individual organisms.

The physiological condition discriminator parameter generating apparatus 1100 comprises a first machine learning unit 1108 that learns a pattern of an attribute of a physiological condition and discrete data included in prulal subdata sets by machine learning. The first machine learning unit 1108 obtains prulal first discriminators that differ from each other, which discriminates the attribute of a physiological condition of each individual included in the subdata set based on the discrete data.

The physiological condition discriminator parameter generating apparatus 1100 comprises a second machine learning unit 1110 that learns a pattern of an attribute of a physiological condition and contiguous data included in prulal subdata sets by machine learning. This second machine learning unit 1110 obtains prulal second discriminators that differ from each other, which discriminates an attribute of a physiological condition of each individual included in the subdata set based on the contiguous data. The physiological condition discriminator parameter generating apparatus 1100 also comprises an outputting unit 1111 that outputs the first discriminator and the second discriminator.

The physiological condition discriminator parameter generating apparatus 1100 also comprises an operator 1124 such as a keyboard, a mouse and/or the like, and a display 1122 such as a liquid crystal display and/or the like. This allows a person operating the physiological condition discriminator parameter generating apparatus 1100 to input various data or commands into the physiological condition discriminator parameter generating apparatus 1100, while referencing to graphic data indicated on the display 1122.

The physiological condition discriminator parameter generating apparatus 1100 is also connected via a network 1118 such as Internet, LAN, WAN, or VPN to a server 1126 such as a file server as well as a measuring apparatus 1128 such as a DNA sequencer, a DNA chip, a PCR, an antibody chip or flow cytometry. This allows the physiological condition discriminator parameter generating apparatus 1100 to read out the learning data set and subject data from the server 1126, and to read learning data set and subject data directly from the measuring apparatus 1128 as the results.

The physiological condition discriminator parameter generating apparatus 1100 is also connected to a physiological condition discriminating apparatus 1200 via a network 1119 such as Internet, LAN, WAN, or VPN. The physiological condition discriminator parameter generating apparatus 1100 can output the first discriminator and the second discriminator from the outputting unit 1111, and transfer the first discriminator and the second discriminator to a discriminator parameter acquiring unit 1121 of physiological condition discriminating apparatus 1200.

Using to the physiological condition discriminator parameter generating apparatus 1100, prulal subdata sets are created that are different from each other, the prulal subdata sets constituting a part of the initially obtained learning data set. Two types of discriminators are present for each of the prulal different subdata sets, and a pattern analysis is performed with these two types of discriminators on subject data that are separately acquired from subject individuals. Using the above-described method, a set of two types of discriminators are obtained that can accurately determine an attribute of a physiological condition of a mammal.

On the other hand, the physiological condition discriminating apparatus 1200 of the present embodiment is a apparatus for discriminating an attribute of a physiological condition of a mammalian individual. The physiological condition discriminating apparatus 1200 comprises a discriminator parameter acquiring unit 1121 that acquires the first discriminator and the second discriminator generated by the physiological condition discriminator parameter generating apparatus 1100. The physiological condition discriminating apparatus 1200 also includes a subject data acquiring unit 1104 that acquires subject data consisting of the discrete data and the contiguous data relating to the subject individual including a combination of discrete data relating to a genomic base sequence of the individual and contiguous data relating to an amount of a specific substance in the individual organism, both of which are obtained from the subject individual.

The physiological condition discriminating apparatus 1200 comprises a subject data analyzer 1112 that generates each of the first discrimination result and the second discrimination result of an attribute of a physiological condition of an individual subject a plurality number of times, by pattern analyzing each subject data a plurality number of times using prulal first discriminators and second discriminators. The physiological condition discriminating apparatus 1200 also comprises an integrated determining unit 1114 that integrally determines the most frequently discriminated attribute of a physiological condition in the first discrimination result and the second discrimination result as an attribute of a physiological condition of a individual subject, by integrating the first discrimination result and the second discrimination for each attribute of a physiological condition. The physiological condition discriminating apparatus 1200 also comprises the outputting unit 1116 that outputs the results of the integrated determination.

The physiological condition discriminating apparatus 1200 also comprises an operator 1144 such as a keyboard, a mouse and/or the like, and a display 1220 such as a liquid crystal display and/or the like. This allows a person operating the physiological condition discriminating apparatus 1200 to input various data or commands into the physiological condition discriminating apparatus 1200, while referencing to graphic data indicated on the display 142.

The physiological condition discriminating apparatus 1200 is also connected via a network 1120 such as the Internet, LAN, WAN, and VPN to a display 1130 such as a liquid crystal display, a printer 1132 such as a laser printer or an ink jet printer, and a server 1134 such as a file server. This allows the physiological condition discriminating apparatus 1200 to display the results of integrated determination from outputting unit 1116 on the display 1130 as graphic data, to print with the printer 1134 as graphic data, and to be stored in the server 1132 in various date formats.

According to the physiological condition discriminating apparatus 1200, the two types of discriminators that are generated according to the physiological condition discriminator parameter generating apparatus 1100 are obtained, and the subject data on an individual subject is analyzed about pattern by these two types of discriminators. As a result, two types of discrimination results are obtained for each prulal different subset data on the individual subject, and thus the two types of discrimination results are each subtotaled with respect to the prulal different subdata sets. The attribute of a physiological condition of the largest combined value, which results from totaling and integrating the subtotal calculation results using a suitable calculation formula, is integrally determined as an attribute of a physiological condition of the individual subject. Therefore, the attribute of a physiological condition of a mammal may be allowed to be precisely determined by this apparatus.

As previously mentioned, although the embodiments of the invention have been described with reference to the drawings, these embodiments are exemplary of the present invention, and thus various configurations other than those described above may also be employed.

The analysis method that is employed by the first machine learning unit 108 and the second machine learning unit 110 in the above-described embodiment is specified as principal component analysis, discriminant analysis, or SVM. However, the analysis method is not particularly limited to these three methods, and thus another analysis method may be employed. A factor analysis, a cluster analysis, a multiple regression analysis, and/or the like, may also be preferably employed as a method of multiple classification analysis other than principal component analysis. Or, a decision tree, a Naïve Bayes classifier, an artificial neural network, a Markov chain Monte Carlo method, a Gibbs sampling, a SOM (self-organizing map), and/or the like, may be preferably employed as a pattern acknowledgement or classification method.

In the above embodiment, human glaucoma onset was discriminated. However, the discrimination is not particularly limited to these diseases, and thus it may be preferably used in various discriminations such as the onset, progression and prognosis on a different non-infectious human disease. It may also be preferably used in various discriminations such as the onset, progression and prognosis on a different infectious human disease. Or, it may also be preferably used in the discrimination of an attribute of a physiological condition of a mammal such as a use for livestock or a use for test animals without necessarily being limited to a human disease.

In the present embodiment, a determination was conducted on an attribute of affected and healthy individual in relation to a physiological condition such as the onset of disease. However, the discrimination is not particularly limited to an attribute of a physiological condition. The apparatus described in the above embodiment may be preferably used in a discrimination for various attributes such as an infectious/non-infectious, a progressive type/a non-progressive type, a favorable prognosis/an unfavorable prognosis of a physiological condition. A similar determination with almost same accuracy is possible even for the infection/the non-infection, the progressive type/the non-progressive type, the favorable prognosis/the non-favorable prognosis as affected/healthy as an attribute of a physiological condition included in a learning data set that is used in the above embodiment.

### Examples

Now the present invention will be described in detail with reference to the following non-limiting Examples.

### Example 1

### Diagnosis of Glaucoma onset by the present integrated determination method using genotype data and cytokine data

Glaucoma is one of the leading causes of blindness, and genetic factors and acquired environmental factors are considered to play a role in its onset. The diagnostic performance of the present method was examined on a typical glaucoma, primary open-angle glaucoma (POAG) using genotype data that is genetic information and cytokine data that reflects an acquired condition of a living organism.

### Samples used

For two independent data sets, 42 POAG samples and 42 healthy control samples were prepared for stage 1, and 73 POAG samples and 53 healthy control samples were prepared for stage 2, respectively. All samples contained genotype data and cytokine data. The stage 1 samples were used for characterization of the disease with machine learning followed by a diagnosis of the stage 2 with this result.

### Selection of SNPs used for genotype data

For this experiment, single nucleotide polymorphisms (SNPs) were selected according to the data previously published by the present inventors (Nakano, et. al: Proc Natl Acad Sci. 2009;106(31):12838-42). Specifically, in the first stage, the complete genome from 418 POAG samples and 300 healthy control samples were analyzed on GeneChip® Human Mapping 500K Array chip (Affy 500k) (Affymetrix, Inc.), and 255 SNPs (p < 0.01) thought to be significant were extracted after a chi-square test on the quality-controlled 331,838 SNPs. In the following second stage, an additional analysis for the SNPs extracted in the first stage was performed on 409 POAG samples and 448 healthy control samples using a custom chip (iSelect) with an iSelect™ Custom Infinium™ Genotype system (Illumina, Inc.) In the final stage, a combination analysis was performed on the data from the above two stages, and those with p-value of < 0.01 in Cochran-Mantel-Haenszel chi-square test and p-value of ≧ 0.05 in Heterogeneity (Cochran's Q test) chi-square test were extracted to obtain 40 SNPs, which were suspected of strong correlation with POAG. Among all the combinations of SNPs, those determined to be D' > 0.9 by Haploview 4.1 (linkage disequilibrium analysis software) were considered to belong to the same LD block and excluded to prevent a possible malfunction in analysis. Ultimately, 29 SNPs were selected as an analysis target. These SNPs were the ones patented by the present inventors (WO 2008/30008).

### Selection of cytokine items used for cytokine data

In order to obtain the cytokine data that is used in the present integrated determination method, blood cytokine concentration data was separately obtained in two stages on a Cytometric Bead Array (CBA) Flex Set System (Becton, Dickinson and Company) that could measure prulal cytokines simultaneously. In the first stage, blood cytokine concentration data was measured on 42 POAG samples and 42 healthy control samples for total of 29 items, including: IL-1β, IL-2, IL-3, IL-4, IL-5, IL-6, IL-7, IL-8, IL-9, IL-10, IL-12P70, IL-13, MCP-1(CCL2), MIP-1α(CCL3), MIP-1β(CCL4), RANTES(CCL5), Eotaxin(CCL11), MIG(CXCL9), basic-FGF, VEGF, G-CSF, GM-CSF, IFN-γ, Fas Ligand, TNF, IP-10, angiogenin, OSM, and LT-α, which could be accurately measured simultaneously by the CBA. From the result, 7 items for which 5% or more of the samples failed to be measured were excluded, 14 items for which 5% or more of the samples had a measurement value of 0.0 were excluded, 5 items which had 5% or higher p-value in a t-test for both groups were excluded, and ultimately narrowed down to three items. These three items were thought to be useful in the diagnosis and were measured in the following second stage on freshly prepared 73 POAG samples and the 52 healthy control samples. The samples used in the cytokine data acquisition were same as those used in the present test.

### Preprocessing of test

For the genotype data of SNPs used in the analysis, missing values were corrected and digitized as a discrete value with reference to a normalization technique for each individual based on SNP allele frequency (see Price, et al: Nat Genet. 2006 Aug; 38(8):904-9). The cytokine data was also independently standardized with a unique standardization method that employed the blood cytokine concentration from the healthy control as a reference. The data was entered into various types of library software as well as statistical processing software "R". The developer of "R" was "R Development Core Team" and the version used was "2.10.1". The version of library "e1071" employed in SVM was 1.5-22 (same for the other Examples described hereafter).

### Test method

From 42 samples each from the POAG and healthy control groups in the stage 1, 20 samples each were randomly sampled and the characteristics of genotype data were learned by machine learning using "Support Vector Machine (SVM)", a standard component of the "e1071" library of "R". Using SVM, 73 POAG samples and 52 healthy control samples were each determined for glaucoma positive or negative in the stage 2, and the determination result was stored. After a series of operations was repeated for 501 times, the same operation was also repeated 500 times on the cytokine data. Finally, a total of 1001 results were obtained for each samples of stage 2, and a majority decision was made by adding up the respective positive or negative determination frequencies for each sample to specifying the majority determination as the final determination of each sample.

### Evaluation of results

Discrimination results thus compiled are shown in Table 1 below.

**Table 1**

| | Genotype data only | Cytokine data only | Present integrated determination method |
|---|---|---|---|
| Diagnosis rate | 67.2% | 67.2% | 74.4% |
| Sensitivity | 67.1% | 67.2% | 79.5% |
| Specificity | 67.3% | 63.5% | 67.3% |

As can be clearly seen in the above Table 1, the diagnosis rate by the present integrated determination method was better than the result obtained by separately diagnosing genotype data and cytokine data.

### Example 2

### Diagnosis of glaucoma progression by present integrated determination method using genotype data and cytokine data

There are two types of glaucoma, progressive and non-progressive types. The present method can be examined for its diagnostic performance with respect to a progressive type and a non-progressive type of glaucoma using genotype data, i.e., genetic information and cytokine data, that reflects an acquired condition of a living organism.
The definition of "progressive type" and "non-progressive type" attributes of a physiological condition is as follows:
"progressive type" includes particularly rapid progression of a certain disease among affected individuals; and
"non-progressive type" includes case of not "progressive type" of a certain disease among affected individuals.

### Samples for use

Similarly to the Example 1, several tens of samples each of the progressive type glaucoma and non-progressive type glaucoma were prepared for stage 1; and several tens of samples each of the progressive type glaucoma and non-progressive type glaucoma were prepared for stage 2, as two independent data sets. All the samples contained genotype data and cytokine data. The stage 1 samples were used for characterization of the disease with machine learning followed by a diagnosis of the stage 2 with this result.

### Selection of SNPs used for genotype data

As in the Example 1, single nucleotide polymorphisms (SNPs) for discrimination were selected. Specifically, in the first stage, the complete genome from several hundreds of the progressive type samples and several hundreds of the non-progressive type samples were analyzed on GeneChip® Human Mapping 500K Array chip (Affy 500k) (Affymetrix, Inc.), and SNPs (p < 0.01) thought to be significant were extracted after a chi-square test on the quality-controlled SNPs. In the following second stage, an additional analysis for the SNPs extracted in the first stage was performed on several hundreds of the progressive type samples and several hundreds of the non-progressive type samples using a custom chip (iSelect) with an iSelect™ Custom Infinium™ Genotype system (Illumina, Inc.). In the final stage, a combination analysis was performed on the data from the above two stages, and those with p-value of < 0.01 in Cochran-Mantel-Haenszel chi-square test and p-value of ≧ 0.05 in Heterogeneity (Cochran's Q test) chi-square test were extracted to obtain SNPs, which were suspected of strong correlation with progressive type glaucoma. Among all combinations of SNPs, those that were determined to be D' > 0.9 by Haploview 4.1 (linkage disequilibrium analysis software) were considered to belong to the same LD block and excluded to prevent a possible malfunction in analysis. Ultimately, several tens or fewer of SNPs were preferably selected as an analysis target.

### Selection of cytokine items used in cytokine data

In order to obtain the cytokine data that was used in the present integrated determination method, blood cytokine concentration data was separately obtained in two stages on a Cytometric Bead Array (CBA) Flex Set System (Becton, Dickinson and Company) that could measure prulal cytokines simultaneously. In the first stage, blood cytokine concentration data was measured on several hundreds of the progressive type samples and several hundreds of the non-progressive type samples for total of 29 items, including: IL-1β, IL-2, IL-3, IL-4, IL-5, IL-6, IL-7, IL-8, IL-9, IL-10, IL-12P70, IL-13, MCP-1(CCL2), MIP-1α(CCL3), MIP-1β(CCL4), RANTES(CCL5), Eotaxin(CCL11), MIG(CXCL9), basic-FGF, VEGF, G-CSF, GM-CSF, IFN-γ, Fas Ligand, TNF, IP-10, angiogenin, OSM, and LT-α, which could be accurately measured simultaneously by the CBA. From the result, items for which 5% or more of the samples failed to be measured were excluded, items for which 5% or more of the samples had a measurement value of 0.0 were excluded, items which had 5% or higher p-value in a t-test for both groups were excluded, and ultimately narrowed down preferably to several items. These several items were thought to be useful in the diagnosis and were measured in the following second stage on freshly prepared several hundreds of the progressive type samples and several hundreds of the non-progressive type samples. The samples used in the cytokine data acquisition were the same as those used in the present test.

### Preprocessing of test

For the genotype data of SNPs used in the analysis, missing values were corrected and digitized as a discrete value as in the Example 1. The cytokine data was also independently standardized with a unique standardization method that employed the blood cytokine concentration from the non-progressive type glaucoma as a reference. The data was entered into various types of library software and statistical processing software "R".

### Test method

From several tens of samples each from the progressive type and the non-progressive type groups in the stage 1, 20 samples each were randomly sampled and the characteristics of the genotype data were learned by machine learning using "Support Vector Machine (SVM)", a standard component of the "e1071" library of "R". Using SVM, the progressive type and the non-progressive type samples were each determined for glaucoma positive or negative in the stage 2, and the determination result was stored. After a series of operations was repeated 501 times, the same operation was also repeated 500 times on the cytokine data. Finally, a total of 1001 results were obtained for each samples of stage 2, and majority decision was made by adding up the respective positive or negative determination frequencies for each sample to specify the majority determination as the final determination of each sample.

The present invention is described with reference to the above Examples. The examples are for illustrative purposes only. Accordingly, one of ordinary skill in the would understand that that various modifications are possible, and included within the scope of the present invention.

In the above-described Examples, discrimination was conducted on an affected or healthy attribute on a physiological condition such as the onset of glaucoma, and discrimination was conducted on a progressive type or non-progressive type of attribute on a physiological condition such as the progression of glaucoma. However, the discrimination is not particularly limited to these attributes of a physiological condition. In other words, similarly to the case of the above-described Examples, a discrimination on various attributes such as an infectious/non-infectious, a progressive type/a non-progressive type, a favorable prognosis/a non-favorable prognosis of a physiological condition such as another infection, or prognosis. A similar determination with almost same accuracy is possible even for the infection/the non-infection, the progressive type/the non-progressive type, the favorable prognosis/the non-favorable prognosis as affected/healthy as an attribute of a physiological condition included in a learning data set that is used in the above Example.

### Reference Signs List

- 102: learning data set acquiring unit
- 104: subject data acquiring unit
- 106: resampler
- 108: first machine learning unit
- 110: second machine learning unit
- 112: subject data analyzer
- 114: integrated determining unit
- 116: outputting unit
- 118: network
- 120: network
- 122: image display
- 124: operating unit
- 126: server
- 128: measurement apparatus
- 130: image display
- 132: printer
- 134: server
- 202: first discriminator parameter acquiring unit
- 204: second discriminator parameter acquiring unit
- 206: optimal analysis method applier
- 208: statistical analysis engine storage
- 210: principal component analysis engine
- 212: discriminant analysis engine
- 214: SVM engine
- 216: converted subject data acquiring unit
- 218: discriminator applier
- 220: first discrimination result generator
- 222: second discrimination result generator
- 302: first discrimination result acquiring unit
- 304: second discrimination result acquiring unit
- 306: subtotal calculator
- 308: first subtotal calculator
- 310: second subtotal calculator
- 312: weight parameter applier
- 314: total calculator
- 316: physiological condition determiner
- 318: integrated parameter storage
- 320: weight parameter database
- 322: integrated calculation formula database
- 324: random parameter generator
- 326: test sample data acquiring unit
- 328: sample subtotal calculator
- 330: sample total calculator
- 332: sample integrated determining unit
- 334: weight parameter selector
- 401: data converter
- 402: genotype data converter
- 404: learning data set conversion formula acquiring unit
- 410: converter
- 412: cytokine data converter
- 414: control group data extractor from subject data set
- 420: extracted data processor
- 500: output data generator
- 502: individual subject identifying data generator
- 504: integrated determination data generator
- 506: predicted determination accuracy data generator
- 508: image data generator
- 602: first statistical analyzer
- 606: first accuracy verifier
- 614: first statistical analysis method selector
- 616: first discriminator parameter generator
- 702: second statistical analyzer
- 706: second accuracy verifier
- 714: second statistical analysis method selector
- 716: second discriminator parameter generator
- 802: genotype data digitizer
- 804: numerical converter
- 806: risk allele data storage
- 808: allele frequency calculator
- 810: normalizer
- 812: cytokine data standardizer
- 814: control group data extractor
- 816: Log converter
- 818: normality determiner
- 820: standardizer
- 902: random extractor
- 904: extraction counter
- 906: test sample extractor
- 1000: physiological condition discriminating apparatus
- 1100: physiological condition discriminator parameter generating apparatus
- 1102: learning data set acquiring unit
- 1104: subject data acquiring unit
- 1106: resampler
- 1108: first machine learning unit
- 1110: second machine learning unit
- 1111: outputting unit
- 1112: subject data analyzer
- 1114: integrated determining unit
- 1116: outputting unit
- 1118: network
- 1120: network
- 1121: discriminator parameter acquiring unit
- 1122: image display
- 1124: operating unit
- 1126: server
- 1128: measurement apparatus
- 1130: image display
- 1132: server
- 1134: printer
- 1142: image display
- 1144: operating unit

## Claims

1. An apparatus for discriminating an attribute of a physiological condition of a mammalian individual, comprising:
a learning data set acquiring unit that acquires a learning data set, wherein the data set relates to a group of individuals consisting of prulal individuals used in machine learning, the group of individuals is obtained from a parent population consisting of individuals belonging to the same species as the subject individual, and the data set includes a combination of the attribute of a physiological condition of the individual, discrete data relating to a genomic base sequence of the individual, and contiguous data relating to an amount of a specific substance in the individual organism;
a resampler that extracts a subdata set, wherein the subdata set relates to prulal subgroups of individuals that differ from each other, the subdata set is obtained by random resampling from the learning data set, and the subdata set includes a combination of the attribute of a physiological condition of each of the individuals included in the subgroups of individuals, the discrete data relating to a genomic base sequence of each of individuals, and the contiguous data relating to an amount of a specific substance in each of the individual organisms;
a first machine learning unit that learns a pattern of the attribute of a physiological condition and the discrete data included in the prulal subdata sets by machine learning to obtain prulal first discriminators that differ from each other, wherein the prulal first discriminators are configured for discriminating the attribute of a physiological condition of each individual included in the subdata set based on the discrete data;
a second machine learning unit that learns a pattern of the attribute of a physiological condition and the contiguous data included in the prulal subdata sets by machine learning to obtain prulal second discriminators that differ from each other, wherein the prulal second discriminators are configured for discriminating the attribute of a physiological condition of each individual included in the subdata set based on the contiguous data;
a subject data acquiring unit that acquires subject data consisting of the discrete data and the contiguous data relating to the subject individual including a combination of the discrete data relating to a genomic base sequence of the individual and the contiguous data relating to an amount of a specific substance in the individual organism, both of which are obtained from the subject individual;
a subject data analyzer that analyzes each of the patterns of the subject data multiple times using the prulal first discriminators and second discriminators, and generates each of a first discrimination result and a second discrimination result of the attribute of physiological condition of the subject individual multiple times;
an integrated determining unit that integrates the first discrimination result and the second discrimination result for each attribute of a physiological condition, and integrally determines the most frequently discriminated attribute of a physiological condition in the first discrimination result and the second discriminatrion result as the attribute of a physiological condition of the individual subject; and
an outputting unit that outputs the result of the integrated determining unit.

2. The apparatus according to claim 1, wherein the discrete data is data relating to a gene polymorphism or a variant.

3. The apparatus according to claim 2, wherein the discrete data is data on a SNP.

4. The apparatus according claim 2 or 3, wherein the discrete data is data that is normalized for each individual based on the gene polymorphism or an SNP allele frequency.

5. The apparatus according to any of claims 1 to 4, wherein the discrete data is data derived from an analysis result from a DNA sequencer, a DNA microarray or a nucleic acid amplification method.

6. The apparatus according to any of claims 1 to 5, wherein the contiguous data is data relating to a blood cytokine concentration of the individual.

7. The apparatus according to claim 6, wherein the cytokine is at least one cytokine selected from the group consisting of IL-1β, IL-2, IL-3, IL-4, IL-5, IL-6, IL-7, IL-8, IL-9, IL-10, IL-12P70, IL-13, MCP-1(CCL2), MIP-1α(CCL3), MIP-1β(CCL4), RANTES(CCL5), Eotaxin(CCL11), MIG(CXCL9), b-FGF, VEGF, G-CSF, GM-CSF, IFN-γ, Fas L, TNF, IP-10, angiogenin, OSM, and LT-α.

8. The apparatus according to claim 6 or 7, wherein the contiguous data comprises a normality determiner that transforms the blood cytokine concentration for each type of cytokine into Log form, determines a normality of an original value and a Log value, and employs a value closer to a normal distribution.

9. The apparatus according to any of claims 6 to 8, wherein the contiguous data is data derived from a blood analysis result of the individual obtained by flow cytometry that uses either an antibody chip having a antibody array that specifically binds to the cytokine or a bead set bound to an antibody that specifically binds to the cytokine.

10. The apparatus according to any of claims 1 to 9, wherein the learning data set acquiring unit is configured to read out the learning data set from the parent population database that stores the learning data set relating to the individual group that is provided inside or outside the apparatus.

11. The apparatus according to claim 10, wherein the parent population database is configured so that a combination of an attribute of a physiological condition of the new individual belonging to the same species as the subject individual, discrete data relating to a genomic base sequence of the new individual, and contiguous data relating to an amount of a specific substance in the new individual organism is added and updated as needed.

12. The apparatus according to any of claims 1 to 11, wherein the resampler includes a random extractor that randomly extracts the subdata set from the learning data set.

13. The apparatus according to claim 12, wherein the resampler includes an extraction counter that controls an extraction process by the random extractor to be repeated for a predetermined number of times greater than or equal to 10 times.

14. The apparatus according to claim 12 or 13, wherein the resampler includes a test sample extractor for extracting test sample data in order to verify the discrimination accuracy of the attribute of a physiological condition according to the first discriminator and/or the second discriminator.

15. The apparatus according to any of claims 1 to 14, wherein the first machine learning unit includes a first statistical analyzer that performs at least one statistical analysis method selected from the group consisting of a principal component analysis, a discriminant analysis, an SVM, a factor analysis, a cluster analysis, a multiple regression analysis, a decision tree, Naïve Bayes classifier, an artificial neural network, a Markov chain Monte Carlo method, a Gibbs sampling, and a SOM.

16. The apparatus according to claim 15, wherein the first statistical analyzer is configured to perform at least one statistical analysis method selected from the group consisting of a principal component analysis, a discriminant analysis, and an SVM.

17. The apparatus according to claim 15 or 16, wherein the first machine learning unit includes a first accuracy verifier that verifies the discrimination accuracy of a sample analysis result obtained by analyzing a pattern of the test sample data randomly extracted from the learning data set using the first discriminator.

18. The apparatus according to claim 17, wherein the first machine learning unit includes a first statistical analysis method selector employing a statistical method with the maximum discrimination accuracy from at least one of the statistical methods based on a verification result according to the first accuracy determiner.

19. The apparatus according to any of claims 1 to 18, wherein the second machine learning unit includes a second statistical analyzer that performs at least one statistical analysis method selected from the group consisting of a principal component analysis, a discriminant analysis, an SVM, a factor analysis, a cluster analysis, a multiple regression analysis, a decision tree, Naïve Bayes classifier, an artificial neural network, a Markov chain Monte Carlo method, a Gibbs sampling, and a SOM.

20. The apparatus according to claim 19, wherein the second statistical analyzer is configured so as to perform at least one statistical analysis method selected from the group consisting of a principal component analysis, a discriminant analysis, and an SVM.

21. The apparatus according to claim 20, wherein the second machine learning unit includes a second accuracy verifier that verifies the discrimination accuracy of a sample analysis result obtained by analyzing a pattern of the test sample data randomly extracted from the learning data set using the second discriminator.

22. The apparatus according to claim 21, wherein the second machine learning unit includes a second statistical analysis method selector employing a statistical method with the maximum discrimination accuracy from at least one of the statistical methods based on a verification result according to the second accuracy determiner.

23. The apparatus according to any of claims 1 to 22, wherein the subject data acquiring unit is configured to obtain subject data relating to the subject individual, including a combination of the discrete data relating to a gene polymorphism of the individual and the contiguous data relating to a blood cytokine concentration of the individual.

24. The apparatus according to claim 23, wherein the subject data acquiring unit includes a data converter that digitizes and/or normalizes the subject data by a method similar to that for the learning data set.

25. The apparatus according to any of claims 1 to 24, wherein the subject data analyzer includes an optimal analysis method applier that respectively uses a statistical analysis method with a maximum degree of discriminant accuracy from at least one statistical analysis method selected from the group consisting of a principal component analysis, a discriminant analysis, an SVM, a factor analysis, a cluster analysis, a multiple regression analysis, a decision tree, Naïve Bayes classifier, an artificial neural network, a Markov chain Monte Carlo method, a Gibbs sampling, and a SOM, as the prulal first discriminators and second discriminators.

26. The apparatus according to claim 25, wherein the optimal analysis method applier is configured to perform at least one statistical analysis method selected from the group consisting of a principal component analysis, a discriminant analysis, and an SVM.

27. The apparatus according to any of claims 1 to 26, wherein the subject data analyzer includes a discriminator applier that analyzes a pattern of the data of the subject by using at least one time each of the prulal first discriminators and second discriminators which are different from each other, and generates the first discrimination result and the second discrimination result of the attribute of a physiological condition of the subject individual.

28. The apparatus according to any of claims 1 to 27, wherein the integrated determining unit comprises:
a subtotal calculator that respectively subtotals the number of times that the subject data in the first discrimination result and the second discrimination result is discriminated as a predetermined attribute of a physiological condition; and
a total calculator that calculates a total of the subtotal results in the first discrimination result and the second discrimination result for each attribute of the physiological condition.

29. The apparatus according to claim 28, wherein the integrated determining unit further comprises a weight parameter applier for calculating the total after weighting by each predetermined parameter in the subtotal result of the first discrimination result and the second discrimination result.

30. The apparatus according to claim 29, wherein the integrated determining unit comprises:
a sample subtotal calculator that acquires a sample subtotal calculation result of the sample analysis result obtained by the subject data analyzer that processes the test sample data that is randomly extracted from the learning data set;
a random parameter generator that randomly generates the weight parameter several times;
a sample total calculator that calculates a total of the sample subtotal results for each attribute of the physiological condition after weighting by the random weight parameter;
a sample integrated determining unit that integrally determines the most discriminated attribute of a physiological condition for each sample individual included in the test sample data in the sample total result as the attribute of a physiological condition of the sample individuals; and
a weight parameter selector that adds up for each weight parameter a determination accuracy of integrated determination result of each of the sample individuals, and employs the weight parameter with maximum determination accuracy.

31. The apparatus according to any of claims 1 to 30, wherein the outputting unit is configured to output together:
information for identifying the subject individual,
the result of the integrated determination, and
a predicated determination accuracy.

32. The apparatus according to any of claims 1 to 31, wherein the mammal is a human.

33. The apparatus according to claim 32, wherein the subject individual is a patient seeking for an advice at a medical institution.

34. A method for discriminating an attribute of a physiological condition of a mammalian individual, comprising:
acquiring a learning data set, wherein the data set relates to a group of individuals consisting of prulal individuals used in a machine learning, the group of individuals is obtained from a parent population consisting of individuals belonging to the same species as the subject individual, and the data set includes a combination of an attribute of a physiological condition of the individual, discrete data relating to a genomic base sequence of the individual, and contiguous data relating to an amount of a specific substance in the individual organism;
extracting a subdata set, wherein the subdata set relates to prulal subgroups of individuals that differ from each other, the subdata set is obtained by random resampling from the learning data set, and the subdata set includes a combination of the attribute of a physiological condition of each individual included in the subgroups of individuals, the discrete data relating to a genomic base sequence of the each individual, and the contiguous data relating to an amount of a specific substance in the each individual organism;
learning a pattern of the attribute of a physiological condition and a discrete data included in the prulal subdata sets by machine learning to obtain prulal first discriminators that differ from each other, the prulal first discriminators for discriminating the attribute of a physiological condition of each individual included in the subdata set based on the discrete data;
learning a pattern of an attribute of a physiological condition and contiguous data included in the prulal subdata sets by machine learning to obtain prulal second discriminators that differ from each other, the prulal second discriminators for discriminating an attribute of a physiological condition of each individual included in the subdata set based on the contiguous data;
acquiring subject data on the subject individual including a combination of the discrete data relating to a genomic base sequence of the individual and the contiguous data relating to an amount of a specific substance in the individual, both of which are obtained from the subject individual;
analyzing each of the patterns of the subject data multiple times using the prulal first discriminators and second discriminators, and generates each of a first discrimination result and a second discrimination result of the attribute of physiological condition of the subject individual multiple times;
integrating the first discrimination result and the second discrimination result for each attribute of a physiological condition, and integrally determining the most frequently discriminated attribute of a physiological condition in the first discrimination result and the second discrimination result as the attribute of a physiological condition of the individual subject; and
outputting the result of the integrated determining unit.

35. An apparatus that generates a discriminator that is used in the method according to claim 34, comprising:
a learning data set acquiring unit that acquires a learning data set, wherein the data set relates to a group of individuals consisting of prulal individuals used in machine learning, the group of individuals is obtained from a parent population consisting of individuals belonging to the same species as the subject individual, and the data set includes a combination of an attribute of a physiological condition of the individual, discrete data relating to a genomic base sequence of the individual, and contiguous data relating to an amount of a specific substance in the individual organism;
a resampler that extracts a subdata set, wherein the subdata set relates to prulal subgroups of individuals that differ from each other, the subdata set is obtained by random resampling from the learning data set, and the subdata set includes a combination of the attribute of a physiological condition of each individual included in the subgroups of individuals, the discrete data relating to a genomic base sequence of the each individual, and the contiguous data relating to an amount of a specific substance in the each individual organism;
a first machine learning unit that learns a pattern of the attribute of a physiological condition and the discrete data included in the prulal subdata sets by machine learning to obtain prulal first discriminators that differ from each other, the prulal first discriminators for discriminating the attribute of a physiological condition of each individual included in the subdata set based on the discrete data;
a second machine learning unit that learns a pattern of the attribute of a physiological condition and the contiguous data included in the prulal subdata sets by machine learning to obtain prulal second discriminators that differ from each other, the prulal second discriminators is configured for discriminating the attribute of a physiological condition of each individual included in the subdata set based on the contiguous data; and
an outputting unit that outputs the first discriminator and the second discriminator.

36. A apparatus for discriminating an attribute of a physiological condition of a mammalian individual, comprising:
a discriminator parameter acquiring unit that obtains the first discriminator parameter and the second discriminator parameter generated by the apparatus of claim 35;
a subject data acquiring unit that acquires subject data consisting of discrete data and contiguous data relating to the subject individual including a combination of discrete data relating to a genomic base sequence of the individual and contiguous data relating to an amount of a specific substance in the individual organism, both of which are obtained from the subject individual;
a subject data analyzer that analyzes each of the patterns of the subject data multiple times using the prulal first discriminators and second discriminators, and generates each of a first discrimination result and a second discrimination result of the attribute of a physiological condition of the subject individual multiple times;
an integrated determining unit that integrates the first discrimination result and the second discrimination result for each attribute of a physiological condition, and integrally determines the most frequently discriminated attribute of a physiological condition in the first discrimination result and the second discrimination result as the attribute of a physiological condition of the individual subject; and
an outputting unit that outputs the result of the integrated determining unit.

37. A program to discriminate an attribute of a physiological condition of a mammalian individual, for causing a computer to:
acquire a learning data set, wherein the data set relates to a group of individuals consisting of prulal individuals used in a machine learning, the group of individuals is obtained from a parent population consisting of individuals belonging to the same species as the subject individual, and the data set includes a combination of an attribute of a physiological condition of the individual, discrete data relating to a genomic base sequence of the individual, and contiguous data relating to an amount of a specific substance in the individual organism;
extract a subdata set, wherein the subdata set relates to prulal subgroups of individuals that differ from each other, the subdata set is obtained by random resampling from the learning data set, and the subdata set includes a combination of the attribute of a physiological condition of each individual included in the subgroups of individuals, the discrete data relating to a genomic base sequence of each of the individuals, and the contiguous data relating to an amount of a specific substance in each of the individual organisms;
learn a pattern of the attribute of a physiological condition and the discrete data included in the prulal subdata sets by machine learning to obtain prulal first discriminators that differ from each other, the prulal first discriminators for discriminating the attribute of a physiological condition of each individual included in the subdata set based on the discrete data;
learn a pattern of an attribute of a physiological condition and contiguous data included in the prulal subdata sets by machine learning to obtain prulal second discriminators that differ from each other, the prulal second discriminators for discriminating the attribute of a physiological condition of each individual included in the subdata set based on the contiguous data;
acquire subject data on the subject individual including a combination of the discrete data relating to a genomic base sequence of the individual and the contiguous data relating to an amount of a specific substance in the individual organism, both of which are obtained from the subject individual;
analyze each of the patterns of the subject data multiple times using the prulal first discriminators and second discriminators, and generates each of a first discrimination result and a second discrimination result of the attribute of physiological condition of the subject individual multiple times;
integrate the first discrimination result and the second discrimination result for each attribute of a physiological condition, and integrally determine the most frequently discriminated attribute of a physiological condition in the first discriminator and the second discriminator as the attribute of a physiological condition of the individual subject; and
output the result of the integrated determining unit.
